# EUROPEAN PATENT SPECIFICATION

(11) **EP 3 955 942 B1**
(45) Date of publication and mention of the grant of the patent: **09.10.2024**
(21) Application number: 20723293.5
(22) Date of filing: 16.04.2020
(51) Int. Cl.: A61K 35/747, A23L 33/135, A61P 19/02

(54) **PROBIOTIC COMPOSITIONS AND USES THEREOF**
PROBIOTISCHE ZUSAMMENSETZUNGEN UND VERWENDUNGEN DAFÜR
COMPOSITIONS PROBIOTIQUES ET UTILISATIONS ASSOCIÉES

(30) Priority: 16.04.2019 GB 201905386
(43) Date of publication of application: 23.02.2022
(73) Proprietor: Probi AB, 223 70 Lund (SE)
(72) Inventor: SOPHOCLEOUS, Antonia, 2054 Nicosia (CY); RALSTON, Stuart Hamilton, Edinburgh EH4 2XU (GB)
(74) Representative: Potter Clarkson
(86) International application number: PCT/EP2020/060768
(87) International publication number: WO 2020/212528

(56) References cited:
- WO-A1-2007/040446
- WO-A1-2014/163568
- WO-A1-2018/187838
- WO-A1-2019/243168
- AU-A4- 2013 100 230
- KR-A- 20180 103 772
- KR-A- 20180 104 544
- US-A1- 2016 129 056
- AH L L ET AL: "Composition useful for treating immune diseases, comprises first composition comprising Lactobacillus acidophilus, second composition comprising Lactobacillus, Bifidobacterium and Streptococcus, and third composition containing rosavin", WPI / 2017 CLARIVATE ANALYTICS,, vol. 2018, no. 70, 19 September 2018 (2018-09-19), XP002799631
- WAITAYANGKOON P ET AL: "Lactobacillus casei l39 as a potential disease-modifying treatment for knee osteoarthritis by reducing intraarticular inflammation", OSTEOARTHRITIS AND CARTILAGE, ELSEVIER, AMSTERDAM, NL, vol. 28, 1 April 2020 (2020-04-01), XP086141075, ISSN: 1063-4584, [retrieved on 20200420], DOI: 10.1016/J.JOCA.2020.02.745
- DATABASE WPI Week 201870, Derwent World Patents Index; AN 2018-738709, XP002799631
- DATABASE WPI Week 201871, Derwent World Patents Index; AN 2018-765240, XP002799632
- JAE-SEON SO ET AL: "enhances type II collagen/glucosamine-mediated suppression of inflammatory responses in experimental osteoarthritis", LIFE SCIENCES, PERGAMON PRESS, OXFORD, GB, vol. 88, no. 7, 8 December 2010 (2010-12-08), pages 358 - 366, XP028139062, ISSN: 0024-3205, [retrieved on 20101216], DOI: 10.1016/J.LFS.2010.12.013
- SEUNG HOON LEE ET AL: "Lactobacillus acidophilus ameliorates pain and cartilage degradation in experimental osteoarthritis", IMMUNOLOGY LETTERS., vol. 203, 1 November 2018 (2018-11-01), NL, pages 6 - 14, XP055710654, ISSN: 0165-2478, DOI: 10.1016/j.imlet.2018.07.003
- LEI M ET AL: "The effect of probiotic Lactobacillus casei Shirota on knee osteoarthritis: a randomised double-blind, placebo-controlled clinical trial", BENEFICIAL MICROBES, vol. 8, no. 5, 2017, pages 697 - 703, XP009521365
- LUIS VITETTA ET AL: "The Gastrointestinal Microbiome and Musculoskeletal Diseases: A Beneficial Role for Probiotics and Prebiotics", PATHOGENS, vol. 2, no. 4, 14 November 2013 (2013-11-14), pages 606 - 626, XP055711275, DOI: 10.3390/pathogens2040606
- SHAHRAM LAVASANI ET AL: "A Novel Probiotic Mixture Exerts a Therapeutic Effect on Experimental Autoimmune Encephalomyelitis Mediated by IL-10 Producing Regulatory T Cells", PLOS ONE, vol. 5, no. 2, 2 February 2010 (2010-02-02), pages e9009, XP055423115, DOI: 10.1371/journal.pone.0009009
- BARBARA BRODY: "Taking Probiotics Supplements Might Keep Your Bones Strong, Per a New Study", 19 October 2018 (2018-10-19), XP002799633, Retrieved from the Internet <URL:https://creakyjoints.org/news/probiotics-increase-bone-density/> [retrieved on 20200703]
- NANCY WALSH: "ASBMR: Probiotics Mediate Bone Loss in Women", 3 October 2018 (2018-10-03), XP002799634, Retrieved from the Internet <URL:https://www.medpagetoday.com/meetingcoverage/asbmr/75470> [retrieved on 20200707]
- MAHENDRAKUMAR R. DUBEY ET AL: "Probiotics: A Promising Tool for Calcium Absorption", THE OPEN NUTRITION JOURNAL, vol. 12, no. 1, 31 August 2018 (2018-08-31), pages 59 - 69, XP055640172, ISSN: 1874-2882, DOI: 10.2174/1874288201812010059
- PER-ANDERS JANSSON ET AL: "Probiotic treatment using a mix of three Lactobacillus strains for lumbar spine bone loss in postmenopausal women: a randomised, double-blind, placebo-controlled, multicentre trial", THE LANCET RHEUMATOLOGY, vol. 1, no. 3, 1 November 2019 (2019-11-01), pages e154 - e162, XP055708913, ISSN: 2665-9913, DOI: 10.1016/S2665-9913(19)30068-2

## Description

### Technical field of the invention

The present invention relates to a combination of live *Lactobacillus* strains, for use in the treatment and/or prevention of osteoarthritis in a mammal, by treating and/or preventing cartilage damage.

### Background of the invention

Osteoarthritis (OA) is a type of joint disease that may result from breakdown of joint cartilage and underlying bone. Unlike in other types of arthritis, in OA only the joints are typically affected. Damage to the cartilage that covers the ends of bones in a joint allows bones to rub together, causing pain, swelling and decreased motion of the joint. Over time, the joint may also lose its normal shape.

The most common symptoms of OA are joint pain and stiffness. Initially, symptoms may occur only following exercise, but over time they may become constant. Other symptoms may include joint swelling, decreased range of motion, and, when the back is affected, weakness or numbness of the arms and legs. The most commonly involved joints are those near the ends of the fingers, at the base of the thumb, neck, lower back, knee, and hips. Joints on one side of the body are often more affected than those on the other. Usually the symptoms develop over some years. They can affect work and normal daily activities.

Osteoarthritis is the most common form of arthritis, affecting about 237 million people (3.3% of the world population) according to the Global Burden of Disease Study 2015 (GBD 2015 Disease and Injury Incidence and Prevalence Collaborators, 2016, Lancet, 388(10053):1545-1602). Among those over 60 years old, about 10% of males and 18% of females are affected. OA is the cause of about 2% of years lived with disability and becomes more common in both sexes as people become older. Younger people sometimes develop OA after joint injuries.

Causes of OA include previous joint injury, abnormal joint or limb development, and inherited factors. Risk is greater in those who are overweight, have legs of different lengths, or have jobs that result in high levels of joint stress. OA is believed to be caused by mechanical stress on the joint and low-grade inflammatory processes. As cartilage is lost, the underlying bone also becomes affected. As pain may make it difficult to exercise, muscle loss may also occur. Diagnosis is typically based on signs and symptoms, with medical imaging and other tests occasionally used to support or rule out other problems. In contrast to rheumatoid arthritis, which is primarily an inflammatory condition, in osteoarthritis, the joints do not become hot or red.

While osteoarthritis is a degenerative joint disease that may cause gross cartilage loss and morphological damage to other joint tissues, more subtle biochemical changes occur in the earliest stages of osteoarthritis progression. The water content of healthy cartilage is finely balanced by compressive force driving water out and hydrostatic and osmotic pressure drawing water in. Collagen fibres exert the compressive force, whereas the Gibbs-Donnan effect and cartilage proteoglycans create osmotic pressure which tends to draw water in.

However, during onset of osteoarthritis, the collagen matrix becomes more disorganized and there is a decrease in proteoglycan content within cartilage. The breakdown of collagen fibers results in a net increase in water content. This increase occurs because whilst there is an overall loss of proteoglycans (and thus a decreased osmotic pull), it is outweighed by a loss of collagen. Without the protective effects of the proteoglycans, the collagen fibers of the cartilage can become susceptible to degradation and thus exacerbate the degeneration. Inflammation of the synovium (joint cavity lining) and the surrounding joint capsule can also occur, though this is often mild compared to the synovial inflammation that occurs in rheumatoid arthritis. Inflammation can happen as breakdown products from the cartilage are released into the synovial space, and the cells lining the joint attempt to remove them.

Other structures within the joint can also be affected in OA. The ligaments within the joint become thickened and fibrotic and the menisci can become damaged and wear away. Menisci can be completely absent by the time a person undergoes a joint replacement. New bone outgrowths, called "spurs" or osteophytes, can form on the margins of the joints, possibly in an attempt to improve the congruence of the articular cartilage surfaces in the absence of the menisci. The subchondral bone volume increases and becomes less mineralized (hypomineralization). All these changes can cause problems with proper functioning. The pain in an osteoarthritic joint has been related to thickened synovium and to subchondral bone lesions.

Current treatment includes exercise, efforts to decrease joint stress, support groups, and pain medications. Efforts to decrease joint stress include resting and the use of a cane. Weight loss may help in those who are overweight. Pain medications may include paracetamol (acetaminophen) as well as NSAIDs such as naproxen or ibuprofen. Long-term opioid use is generally discouraged due to lack of information on benefits as well as risks of addiction and other side effects. If pain interferes with normal life despite other treatments, joint replacement surgery may help. An artificial joint typically lasts 10 to 15 years.
- KR 2018/0103772 A relates to a composition for preventing and treating immune diseases comprising a mixture of lactic acid bacteria and various applications thereof.
- KR 2018/0104544 A relates to a composition comprising *Lactobacillus acidophilus* for preventing or treating one or more of arthritis, fibrosis, colitis, and transplant rejection, and to various applications thereof.
- So et al., 2011, Life Sci., 88(7): 358-366, relates to *Lactobacillus casei* that is said to enhance type II collagen/glucosamine-mediated suppression of inflammatory responses in experimental osteoarthritis.
- Lee et al., 2018, Immunol. Lett., 203: 6-14, relates to *Lactobacillus acidophilus* that is said to ameliorate pain and cartilage degradation in experimental osteoarthritis.
- Lei et al., 2017, Benef. Microbes, 8(5): 697-703, relates to a random double-blind, placebo-controlled clinical trial that is said to investigate the effect of probiotic *Lactobacillus casei* Shirota on knee osteoarthritis.
- WO 2018/187838 A1 relates to methods for treating or preventing inflammation, or an inflammatory or autoimmune condition, methods for treating or preventing a skin or nail condition or infection, methods for treating or preventing symptoms of gastrointestinal infections, and methods for promoting wound healing.
- AU 2013/100230 A4 relates to compositions and methods for the treatment of rheumatic disorders such as arthritis.
- US 2016/0129056 A1 relates to compositions and uses thereof to aid joint support, aging, and sports medicine recovery and performance that include *Lactobacillus rhamnosus.*
- Vitetta et al., 2013, Pathogens, 2(4): 606-626, relates to a beneficial role for probiotics and prebiotics in the gastrointestinal microbiome and musculoskeletal diseases.
- Lavasani et al., 2010, PLoS ONE, 5(2): e9009, relates to a novel probiotic mixture that exerts a therapeutic effect on experimental autoimmune encephalomyelitis mediated by IL-10 producing regulatory T cells.
- Brody 2018 (https://creakyjoints.org/news/probiotics-increase-bone-density/) is a web article entitled "Taking probiotics supplements might keep your bones strong, per a new study".
- Walsh 2018 (https://medpagetoday.com/meetingcoverage/asbmr/75470) is a web article entitled "ASBMR: probiotics mediate bone loss in women".
- Dubey et al., 2018, The Open Nutrition Journal, 12(1): 59-69, relates to probiotics as a promising tool for calcium absorption.
- WO 2014/163568 A1 relates to at least one probiotic strain chosen from *Lactobacillus paracasei,* or at least one probiotic strain chosen from *Lactobacillus paracasei* in combination with at least one probiotic strain chosen from *Lactobacillus plantarum,* for use in the treatment or prevention of osteoporosis or for use in increasing the absorption of at Ca²⁺ ions, in a mammal, preferably in a human.
- WO 2007/040446 A1 relates to the use of at least one strain of probiotic bacteria selected from *Lactobacillus* for the manufacture of a medicament for the treatment and/or prevention of an autoimmune disease.

Thus, there is still a need within the art to find effective preventive and therapeutic methods against osteoarthritis in a mammal, and particularly in humans.

The present invention seeks to address problems with osteoarthritis.

As shown in the accompanying experimental example, administration of probiotic bacteria according to the invention has surprisingly positive effects against osteoarthritis in mammals by preventing and/or reducing cartilage damage, bone damage at a joint, and inflammation associated with osteoarthritis.

### Description of the invention

The present invention is as defined in the claims. Hence, the invention provides a combination of live *Lactobacillus* strains, for use in the treatment and/or prevention of osteoarthritis in a mammal, wherein the combination of *Lactobacillus* strains is *Lactobacillus paracasei* DSM 13434 (8700:2) and at least one strain of *Lactobacillus plantarum,* and wherein the treatment and/or prevention of osteoarthritis is by treating and/or preventing cartilage damage, preferably wherein the mammal is a human. The live *Lactobacillus* strains are therefore probiotic strains of *Lactobacillus.*

By "use in the treatment and/or prevention" we include the meaning of a use which gives rise to an effect in a subject of preventing, delaying, protecting against, reducing the severity of and/or removing, one or more symptoms and/or other markers associated with osteoarthritis.

Preferably, the treatment and/or prevention of osteoarthritis is further by reducing and/or preventing bone damage at a joint, and/or inflammation associated with osteoarthritis.

Preferably, the use according to the invention is further by treating and/or preventing bone damage at a joint.

Preferably, the use according to the invention is further by treating and/or preventing inflammation associated with osteoarthritis, preferably by treating and/or preventing inflammation localised to a joint.

By "treat", "treatment" or "treating" we include the meaning that the event or condition being treated is ameliorated, reduced in severity, removed, blocked from occurring further, protected against occurring further, delayed and/or made to cease. Such treatment typically takes place after the event (or the same kind of event) has occurred or the condition is manifest. It will also be appreciated that such terms may include the meaning that an event or condition is maintained in the current state without becoming worse or developing further.

By "prevent", "prevention" or "preventing" we include the meaning that the event or condition being prevented is protected against, delayed, reduced (e.g. reduced in severity), blocked from occurring, or made to cease. Such prevention typically takes place before the event occurs or the condition is manifest, but it will be appreciated that it can also mean to prevent further occurrence of the same kind of event. It will also be appreciated that such terms may include the meaning that an event or condition is maintained in the current state without becoming worse or developing further.

For example, a measure of osteoarthritis (e.g. osteoarthritis score, such as according to Glasson et al, 2010, Osteoarthritis Cartilage, 18 Suppl 3:S17-23) following administration of the combination for use according to the invention may be reduced by at least 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 96%, 97%, 98% or at least 99% compared to without administration of the combination, or compared to administration of a corresponding composition lacking the combination. A minimum of one region of one joint should be measured in the mammal. For example, it is preferred that osteoarthritis score is measured in cartilage at the knee joint, such as at the tibia or the femur. Osteoarthritis score at the knee joint may be measured in cartilage at the lateral or the medial side of the knee joint. As shown in the example herein where the medial meniscus was destabilised, osteoarthritis score at the knee joint may particularly be measured in cartilage at the femur, particularly the medial femur. It is also preferable for measures of bone to be made at a contact point (plate) of cortical bones within joints, such as a subchondral bone plate of the knee, and/or in epiphyseal trabecular bone at a joint (including subchondral bone), such as epiphyseal trabecular bone of the tibia or femur at the knee joint.

By "cartilage damage" we include the meaning of any flaw, defect, breakdown, injury, or tear of cartilage at a joint, particularly where such damage could lead to, or is otherwise associated with, osteoarthritis.

Cartilage is a resilient and smooth elastic tissue, a rubber-like padding that covers and protects the ends of long bones at the joints, and is a structural component of the rib cage, the ear, the nose, the bronchial tubes, the intervertebral discs, and many other body components. It is not as hard and rigid as bone, but it is much stiffer and much less flexible than muscle. The matrix of cartilage is made up of glycosaminoglycans, proteoglycans, collagen fibres and, sometimes, elastin.

Cartilage is composed of specialized cells called chondrocytes that produce a large amount of collagenous extracellular matrix, abundant ground substance that is rich in proteoglycan and elastin fibres.

Cartilage is classified in three types, elastic cartilage, hyaline cartilage and fibrocartilage, which differ in relative amounts of collagen and proteoglycan.

Elastic cartilage is present in the outer ear, Eustachian tube and epiglottis, and contains elastic fibre networks, collagen type II fibres, and elastin as the principal protein.

Fibrocartilage consists of a mixture of white fibrous tissue and cartilaginous tissue in various proportions and is the only type of cartilage that contains type I collagen in addition to the normal type II collagen. Fibrocartilage is found in the soft tissue to bone attachments, the pubic symphysis, the anulus fibrosus of intervertebral discs, menisci, the triangular fibrocartilage discus of the wrist and the temporomandibular joints connecting the jawbone to the skull. Fibrocartilage can also sometimes form as a scar in places where hyaline cartilage is torn all the way down to the bone, but is not an ideal replacement for the smooth, glassy articular cartilage that normally covers the surface of joints ('articular' means of, at, or relating to the joints of the body). Although fibrocartilage is able to fill in articular cartilage defects, its structure is significantly different from that of hyaline cartilage; it is much denser and it doesn't withstand the demands of everyday activities as much as hyaline cartilage. Fibrocartilage is therefore at a higher risk of breaking down than hyaline cartilage.

Hyaline cartilage is most prevalent type of cartilage and is found on many joint surfaces (where it is called articular cartilage), as well as in the ribs, nose, larynx and trachea. Hyaline cartilage is primarily made of type II collagen and chondroitin sulphate. Although articular cartilage is often found in close contact with menisci and articular discs, articular cartilage is not considered a part of either of these structure, which are made entirely of fibrocartilage. Hence, it is the biochemical breakdown of articular cartilage that frequently results in osteoarthritis.

Hyaline cartilage is covered externally by a fibrous membrane known as the perichondrium or, when it's along articulating surfaces, the synovial membrane. This membrane contains vessels that provide the cartilage with nutrition through diffusion. Cartilage itself does not contain blood vessels (it is avascular) or nerves (it is aneural). The compression of the articular cartilage or flexion of the elastic cartilage generates fluid flow, which assists diffusion of nutrients to the chondrocytes. Compared to other connective tissues, cartilage has a very slow turnover of its extracellular matrix and does not repair.

Articular cartilage damage in the knee may be found on its own but it will more often be found in conjunction with injuries to ligaments and menisci. People with previous surgical interventions face more chances of articular cartilage damage due to altered mechanics of the joint. Articular cartilage damage may also be found in the shoulder causing pain, discomfort and limited movement. Articular cartilage does not usually regenerate (the process of repair by formation of the same type of tissue) after injury or disease leading to loss of tissue and formation of a defect such as osteoarthritis.

Cartilage damage may be determined by any suitable method known in the art. For example, cartilage damage may be determined by MRI scan or by arthroscopy. Damage may be assessed by a grading system, such as the arthroscopic grading system established by the International Cartilage Repair Society:
grade 0: (normal) healthy cartilage;
grade 1: the cartilage has a soft spot, blisters, or superficial wear;
grade 2: minor tears of less than one-half the thickness of the cartilage layer;
grade 3: lesions have deep crevices of more than one-half the thickness of the cartilage layer;
grade 4: the cartilage tear is full thickness and exposes the underlying (subchondral) bone.

Alternatively, damage may be assessed using the scoring system recommended by the Osteoarthritis Research Society International (OARSI) detailed in Glasson et al (2010, Osteoarthritis Cartilage, 18 Suppl 3:S17-23), particularly when damage is being assessed in mice.

In a preferred embodiment, cartilage damage is assessed at the knee joint, such as at the tibia and/or femur. Cartilage damage may be assessed at the lateral or medial side of the knee joint. For example, cartilage damage may be assessed at the medial tibia, lateral tibia, medial femur and/or lateral femur. Preferably, such as in a clinical context, cartilage damage is assessed at all four of these sites. However, it will be appreciated that it may only be necessary to assess cartilage damage at one, two or three of these sites. As shown in the example herein where the medial meniscus was destablised, cartilage damage may be assessed at the medial side of the knee joint, particularly at the medial femur.

By "bone damage at a joint" we include the meaning of any flaw, defect, breakdown, injury or lesion of bone in the region of a joint, particularly where such damage could lead to, or is otherwise associated with, osteoarthritis. We also include any disease-associated increase in bone volume and hypomineralization that can occur during osteoarthritis. For example, thickening of trabecular epiphyseal bone and cortical subchondral bone plates (contact points) are typical structural changes that may be observed in osteoarthritis.

Bone tissue is a mineralized tissue of two types, cortical (also known as compact) bone and trabecular (also known as cancellous or spongy) bone.

Cortical bone is denser and stronger than trabecular bone and forms the hard exterior (cortex) of bones. Microscopically, cortical bone in humans is composed of osteons, columns formed of concentric rings of calcified matrix called lamellae that surround a central (Haversian) canal containing the blood vessels, nerves and lymphatic vessels. Cortical bone typically has an outer surface of periosteum connective tissue and an inner surface of endosteum connective tissue that forms the boundary between cortical and trabecular bone.

Trabecular bone is the internal tissue of the skeletal bone and is an open cell porous network comprising tiny lattice-shaped units (trabeculae). Trabecular bone has a higher surface-area-to-volume ratio than cortical bone because it is less dense, making it softer and weaker, but more flexible. The greater surface area also makes it suitable for metabolic activities such as the exchange of calcium ions. Trabecular bone is typically found at the ends of long bones, near to joints and within the interior of vertebrae. Microscopically, the primary anatomical and functional unit of trabecular bone is the trabecula. Unlike the concentric circles of the osteons, trabeculae typically form an irregular network of thin rod-like formations of osteoblasts covered in endosteum, and the spaces between are filled with bone marrow and hematopoietic stem cells. Trabecular bone accounts for approximately 20% of total bone mass but has nearly ten times the surface area compared to cortical bone.

Other types of tissue found in bones include bone marrow, endosteum, periosteum, nerves, blood vessels and cartilage.

There are five types of bones in the human (mammalian) body, characterised by their shape: long, short, flat, irregular, and sesamoid. 'Long bones', e.g. the femur and most other bones of the limbs, fingers and toes, typically have a shaft (diaphysis) mostly made of cortical bone that forms a cavity filled with lesser amounts of marrow, and a rounded head (epiphysis) at each end of the bone. An epiphysis typically comprises trabecular bone surrounded by layers of cortical bone. 'Short bones', e.g. those of the wrist and ankle, are roughly cube-shaped and typically comprise a thin layer of cortical bone surrounding an interior of trabecular bone. `Flat bones', e.g. the sternum, ribs, hips and most of the skull bones, are thin and generally curved, with two parallel layers of cortical bone sandwiching a layer of trabecular bone. 'Irregular bones', e.g. vertebrae, sacrum, coccyx, temporal, sphenoid, ethmoid, zygomatic, maxilla, mandible, palatine, inferior nasal concha, and hyoid, have an irregular shape and typically comprise thin layers of compact bone surrounding an interior of trabecular bone. Sesamoid bones, e.g. patella and pisiform, are bones embedded in tendons.

Bone tissue (osseus tissue) of both cortical and trabecular bone typically comprises a relatively small number of cells trapped in a tough matrix of collagen (ossein) fibres on which inorganic salt crystals (e.g. calcium hydroxylapatite / hydroxyapatite) adheres to strengthen the bone. The remainder of the matrix is typically filled with ground substance - an amorphous gel-like substance in the extracellular space that contains all components of the extracellular matrix including water, glycosaminoglycans (GAGs; e.g. hyaluronan), proteoglycans which GAGs are bound to (e.g. heparan sulfate and keratin sulfate), glycoproteins (e.g.osteonectin, osteopontin, bone sialoprotein), osteocalcin, and link proteins (e.g. vinculin, spectrin and actomysin). Bone is formed by the hardening of the matrix around entrapped cells, which typically change from osteoblasts to inactive osteocytes.

The skeleton is remodeled by bone forming osteoblasts (OBs) and bone resorbing osteoclasts (OCLs). Macrophage colony stimulating factor (M-CSF) increases proliferation and survival of OCLs precursor cells as well as up-regulates expression of receptor activator of nuclear factor-κB (RANK) in OCL. This allows RANK ligand (RANKL) to bind and start the signalling cascade that leads to OCL formation. The effect of RANKL can be inhibited by Osteoprotegerin (OPG), which is a decoy receptor for RANKL.

The association between inflammation and bone loss is well established and in autoimmune diseases osteoclastic bone resorption is driven by inflammatory cytokines produced by activated T-cells. In addition, several studies demonstrate that low-grade systemic inflammation, indicated by moderately elevated serum levels of high sensitivity C-reactive protein (hsCRP), associate with low BMD, elevated bone resorption and increased fracture risk. The estrogen deficiency that occurs after menopause results in increased formation and prolonged survival of osteoclasts. This is suggested to be due to a number of factors including loss of the immunosuppressive effects of estrogen, resulting in increased production of cytokines promoting osteoclastogenesis, and direct effects of estrogen on OCLs. In line with these data, blockade of the inflammatory cytokines TNFα and IL-1 leads to a decrease in bone resorption markers in early postmenopausal women.

Bone damage may be determined by any suitable measure known in the art. For example, bone damage may be determined by measuring bone loss, such as change in bone mineral content or bone mineral density. Preferably, bone damage is assessed at a joint, for example, in epiphyseal bone of the knee joint (which includes the subchondral bone), particularly one or both of tibial epiphyseal trabecular bone (trabecular bone at the proximal epiphysis of the tibia) and femoral epiphyseal trabecular bone (trabecular bone at the distal epiphysis of the femur, such as part of one or both of the femoral condyles), one or both of the subchondral medial tibial and femoral bone plates, and/or one or both of the subchondral lateral tibial and femoral bone plates (all four of which are cortical bone).

The upper part of the tibia is called the proximal tibia or proximal tibial epiphysis. According to McKinnis (2014, Fundamentals of Musculoskeletal Imaging, 4th ed, F.A. Davis Company, Philadelphia; particularly Figure 13-1, accessible on 16 April 2019 at https://fadavispt.mhmedical.com/content.aspx?bookid=1899&sectionid=141191793) the proximal tibia consists of medial and lateral condyles, which superiorly form the articular surface of the tibia, or the tibial plateau. Similarly, the lower part of the femur is called the distal femur or distal femoral epiphysis. According to McKinnis (2014, *supra*), the distal femur exhibits medial and lateral condyles (see Figure 13-1 of McKinnis, *supra*).

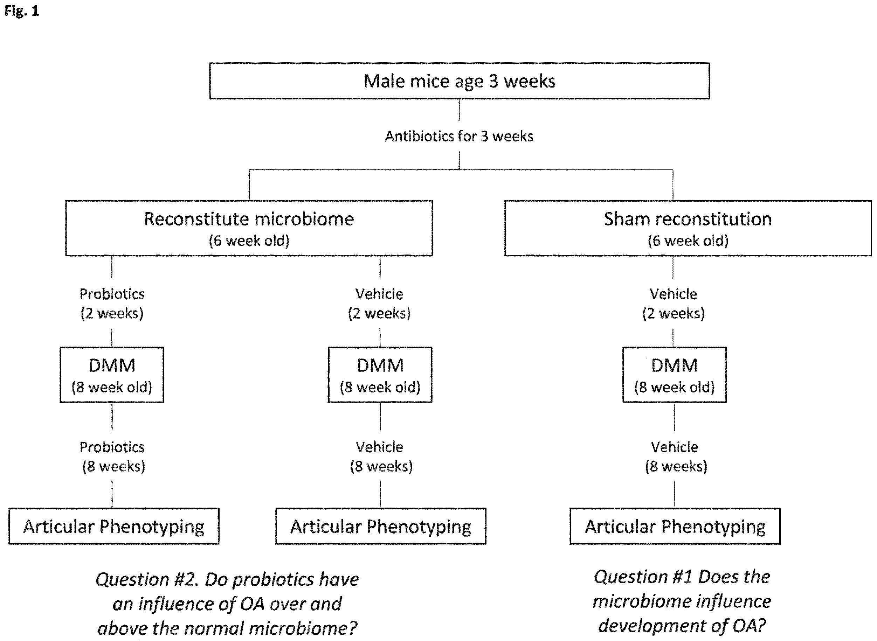

By "subchondral" we include the meaning of the layer of bone just below the cartilage in a joint. Hence, in the knee joint, the subchondral bone of the proximal tibia is just below (distal to) the cartilage of the knee joint, and the subchondral bone of the distal femur is just 'below' (proximal to) the cartilage of the knee joint. Subchondral bone consists of a cortical part (subchondral bone plates) and a trabecular part (subchondral trabecular bone), for example, see Figure 1 of Arijmand et al (2019, Sci Rep 8(1):11478).

By "epiphyseal" we include the meaning of the region of the bone comprising the epiphysis, including subchondral bone. Hence, for bones of the knee joint, e.g. the tibia, "epiphyseal bone" includes the regions named as subchondral and epiphyseal in Figure 1 of Arijmand *et al, supra.*

By "bone mineral content" (BMC) we include the meaning of the amount of bone minerals (e.g. calcium) in bone tissue. Measures of BMC typically include g and g/cm. Preferably BMC is measured in grams (g).

By "bone mineral density" (BMD) we include the meaning of the amount of bone minerals (e.g. calcium) in bone tissue, expressed as a density. Measures of BMD typically include mass of mineral per volume of bone (e.g. cubic centimetre) or, when assessed by clinical imaging (densitometry), optical density per area (e.g. square centimetre) of bone surface. For example, BMD may be expressed in g/cm² or in g/cm³. Hence, BMD may be calculated from BMC in g by dividing by the surface area of the bone, or from BMC in g/cm by dividing by the width of the bone at the scanned line. BMD is typically a measure of the strength of a bone, and reduced bone mineral density may increase the chance of fractures.

Bone mineral content and bone mineral density may be measured by any method known in the art. For example, BMC and BMD may be measured by dual-energy X-ray absorptiometry (DXA or DEXA), dual X-ray absorptiometry and laser (DXL), quantitative computed tomography (QCT), quantitative ultrasound (QUS), single photon absorptiometry (SPA), dual photon absorptiometry (DPA), digital X-ray radiogrammetry (DXR) or single energy X-ray absorptiometry (SEXA). DXA is the most widely used technique but QCT (or X-ray micro-computed tomography [micro-CT] for small animals) is preferred as it is capable of measuring the bone's volume.

By "inflammation associated with osteoarthritis" we include the meaning of any inflammation resulting from the condition of osteoarthritis. Hence, typically, inflammation associated with osteoarthritis is localised to a joint. By "inflammation localised to a joint" we include the meaning of inflammation of the synovium (joint cavity lining) and the surrounding joint capsule. Such inflammation can result from the release of cartilage breakdown products into the synovial space and the attempted removal of those breakdown products by the cells lining the joint. In particular, "inflammation associated with osteoarthritis" and "inflammation localised to a joint" include pannus, synovial hyperplasia and sub-synovial inflammation (see also Table 2, *infra*)*.*

By "pannus" we include the meaning of outgrowth of fibrous tissue, synovium and/or inflammatory cells spreading over the surface of the bone and/or cartilage at the joint margins. By "synovial hyperplasia", also known as synoviocyte hyperplasia, we include the meaning of an increase in cell number of the synovial fibroblasts. By "sub-synovial inflammation" we include the meaning of the infiltration of inflammatory cells (neutrophils, macrophages and/or lymphocytes) into the synovial space.

However, severe chronic inflammation in joints (e.g. leading to heat and redness) is not a typical sign of osteoarthritis, but is normally a sign of rheumatoid arthritis. Hence, the degree of inflammation in osteoarthritis is usually mild compared to rheumatoid arthritis.

Where inflammation is not present but the features of osteoarthritis are present, the condition may be referred to as osteoarthrosis. Nevertheless, according to common usage of the term "osteoarthritis", the broad use herein of the term "osteoarthritis" is also intended to encompass the condition "osteoarthrosis".

### Patient group

The combination for use according to the invention is suitable to be used in mammals. The combination for use according to the invention may be suitable for rodent mammals, e.g. mice, rats, guinea pigs. Preferably, the combination is suitable to be used by non-rodent mammals, e.g. cats, dogs, horses, monkeys. Most preferably, the combination is suitable for humans (men and/or women), including elderly people (such as humans older than 50, 55, 60, 65, 70, 75, 80 or 85 years), post-menopausal women, peri-menopausal women and pre-menopausal women, as these are individuals in which osteoarthritis is or may typically become a problem. The combination for use may also be suitable for obese mammals, especially obese humans (men and/or women).

Otherwise healthy people may also benefit from the invention in order to prevent getting cartilage damage, and preferably also to prevent getting bone damage at a joint, and/or inflammation localised to a joint, each of which can lead to osteoarthritis.

Preferably, the combination for use according to the invention is for use to treat and/or prevent OA in a post-menopausal woman, a woman six years or less after onset of menopause, a peri-menopausal woman, or a pre-menopausal woman.

Preferably, the combination for use according to the invention is effective to treat and/or prevent osteoarthritis in a male mammal, in particular, a man. Preferably, it is for use in a man older than 45, 50, 55, 60, 65, 70, 75 or 80, or a man between the ages of 45 and 80, such as between 45 and 50, 50 and 55, 55 and 60, 60 and 65, 65 and 70, 70 and 75, 75 and 80, 45 and 55, 50 and 60, 55 and 65, 60 and 70, 65 and 75, 70 and 80, 45 and 60, 50 and 65, 55 and 70, 60 and 75, 65 and 80, 45 and 65, 50 and 70, 55 and 75, 60 and 80, 45 and 70, 50 and 75, 55 and 80, 45 and 75, or 50 and 80.

Additionally or alternatively, the combination for use according to the invention is effective to treat and/or prevent osteoarthritis in a female mammal, in particular, a post-menopausal woman, a woman six years or less after onset of menopause, a peri-menopausal woman, or a pre-menopausal woman. In particular, the combination may be suitable for use in a woman older than 45, 50, 55, 60, 65, 70, 75 or 80. For example, the combination may be suitable for use in a woman between the ages of 45 and 80, such as between 45 and 50, 50 and 55, 55 and 60, 60 and 65, 65 and 70, 70 and 75, 75 and 80, 45 and 55, 50 and 60, 55 and 65, 60 and 70, 65 and 75, 70 and 80, 45 and 60, 50 and 65, 55 and 70, 60 and 75, 65 and 80, 45 and 65, 50 and 70, 55 and 75, 60 and 80, 45 and 70, 50 and 75, 55 and 80, 45 and 75, or 50 and 80.

Preferably, a `post-menopausal woman' is a woman within one, two, three, four, five, six, seven, eight, nine, ten, eleven or twelve years from onset of menopause, and most preferably a woman six years or less after onset of menopause.

Menopause is the time in most women's lives when menstrual periods stop permanently, and they are no longer able to bear children. Menopause typically occurs between 49 and 52 years of age. Medical professionals often define menopause as having occurred when a woman has not had any vaginal bleeding for a year. Hence, the date of menopause itself is typically determined retroactively, once 12 months have passed after the last appearance of menstrual blood. At the physiological level, menopause happens because of a decrease in the ovaries' production of the hormones oestrogen and progesterone. Hence, menopause may also be defined by a decrease in the production of these hormones by the ovaries, and a diagnosis of menopause can be confirmed by measuring hormone levels in the blood or urine. In those who have had surgery to remove their uterus but still have ovaries, menopause may be viewed to have occurred at the time of the surgery or when their hormone levels fell. Following the removal of the uterus, symptoms of menopause typically occur earlier.

The term "pre-menopause" includes the meaning of the years leading up to the last menstrual period, when the levels of reproductive hormones are becoming more variable and lower, and the effects of hormone withdrawal are present. Pre-menopause typically starts before the monthly cycles become noticeably irregular in timing. A "pre-menopausal woman" is a woman in her time of pre-menopause.

The term "peri-menopause" (literally 'around the menopause') refers to the menopause transition years and is typically a time before and after the date of the final episode of menstrual flow. Hence, a "peri-menopausal woman" is a woman in her time of peri-menopause. Typically, peri-menopause begins between 40 and 50 years of age (average 47.5 years) and may last for four to ten years. For example, peri-menopause may be four to eight years, beginning with the time of changes in the length of times between periods and ending one year after the final menstrual period (The North American Menopause Society, https://web.archive.org/web/2013041011 1346/http://www.menopause.org/for-women/menopauseflashes/menopause-101-a-prime r-for-the-perimenopausal). Alternatively, peri-menopause may be six to ten years ending 12 months after the last menstrual period (Dr Jerilynn C. Prior, Centre for Menstrual Cycle and Ovulation Research, https://web.archive.org/web/2013022505 5347/http://cemcor.ca/help_yourself/perimenopause). Oestrogen levels average about 20-30% higher during peri-menopause than during pre-menopause, often with wide fluctuations. These fluctuations cause many of the physical changes during perimenopause as well as menopause, including hot flashes, night sweats, difficulty sleeping, vaginal dryness or atrophy, incontinence, osteoporosis, and heart disease. The term "postmenopausal" typically describes women who have not experienced any menstrual flow for a minimum of 12 months (thus confirming that menstrual cycles have ceased), assuming that they have a uterus and are not pregnant or lactating. In women without a uterus, menopause or post-menopause can be identified by a blood test showing a very high level of follicle stimulating hormone (FSH). Thus, post-menopause may also be defined as the time after the point when a woman's ovaries become inactive. As a woman's reproductive hormone levels continue to drop and fluctuate for some time into post-menopause, hormone withdrawal effects such as hot flashes may take several years to disappear.

### Probiotic strains

Probiotic bacteria are defined as "live microorganisms that, when administered in adequate amounts, confer a health benefit on the host" (Hill et al, Nat Rev Gastroenterol Hepatol, 2014, 11(8):506-514). Bacteria of the genera *Lactobacillus* and *Bifidobacterium* are the most frequently used bacteria in probiotic products. These bacteria are generally safe, as are probiotic products based on these organisms. For a bacterium to fulfil the definition of a probiotic it typically has to be able to survive in and colonise the intestines, survive the processes of production and storage, and have evidence that it has positive effects on consumer health.

By "probiotic strains" we include the meaning of strains of bacteria which when administered in adequate amounts confer a health benefit on the host. Typically, the administration of said probiotic strains will alter the composition of the gut microbiota.

In accordance with the present claims, the combination of live *Lactobacillus* strains is *Lactobacillus paracasei* DSM 13434 (8700:2) and at least one strain of *Lactobacillus plantarum.*

Otherwise disclosed herein is at least one probiotic strain for use in the treatment and/or prevention of osteoarthritis in a mammal, wherein the at least one probiotic strain may be chosen from any suitable strain of *Lactobacillus,* in particular from one or more strain(s) of the following species: *Lactobacillus paracasei*, *Lactobacillus plantarum*, *Lactobacillus rhamnosus, Lactobacillus crispatus, Lactobacillus gasseri, Lactobacillus fermentum, Lactobacillus reuteri, Lactobacillus acidophilus, Lactobacillus helveticus, Lactobacillus casei, Lactobacillus salivarius, and Lactobacillus johnsonii.* For example, the at least one probiotic strain of *Lactobacillus* may be at least one strain of *Lactobacillus paracasei,* preferably selected from *Lactobacillus paracasei* DSM 13434 (8700:2) or *Lactobacillus paracasei* DSM 13432 (02:A), preferably *Lactobacillus paracasei* DSM 13434 (8700:2). Alternatively or additionally, the at least one probiotic strain of *Lactobacillus* may be at least one strain of *Lactobacillus plantarum.* For example, the at least one probiotic strain of *Lactobacillus* may be chosen from at least one probiotic strain of *Lactobacillus paracasei* and/or at least one probiotic strain of *Lactobacillus plantarum.* The probiotic strains may be viable, inactivated or dead.

*Lactobacillus paracasei* DSM 13434 (8700:2) and *Lactobacillus paracasei* DSM 13432 (02:A) were deposited on 6 April 2000 at DSMZ-DEUTSCHE SAMMLUNG VON MIKROORGANISMEN UND ZELLKULTUREN GmbH, Mascheroder Weg 1b, D-38124 Braunschweig, Germany, by Probi AB.

Preferably, the at least one strain of *Lactobacillus plantarum* (such as in the combination for use of the claimed invention) is selected from *Lactobacillus plantarum* DSM 6595 (299), *Lactobacillus plantarum* DSM 9843 (299V^{®}), *Lactobacillus plantarum* DSM 15312 (HEAL *9), Lactobacillus plantarum* DSM 15313 (HEAL 19), *Lactobacillus plantarum* DSM 15316 (HEAL 99), *Lactobacillus plantarum* DSM 32131 (GOS42), *Lactobacillus plantarum* DSM 17852 (LB3e) and *Lactobacillus plantarum* DSM 17853 (LB7c), all of which are probiotic strains.

*Lactobacillus plantarum* DSM 6595 (299) was deposited on 2 July 1991 at DSM-DEUTSCHE SAMMLUNG VON MIKROORGANISMEN UND ZELLKULTUREN GmbH, Mascheroder Weg 1 B, D-3300 Braunschweig, Germany, in the name of Probi (i.e. Probi AB).

*Lactobacillus plantarum* DSM 9843 (299V^{®}) was deposited on 16 March 1995 at DSM-DEUTSCHE SAMMLUNG VON MIKROORGANISMEN UND ZELLKULTUREN GmbH, Mascheroder Weg 1b, D-38124 Braunschweig, Germany, by Probi AB.

*Lactobacillus plantarum* DSM 15312 (HEAL 9), *Lactobacillus plantarum* DSM 15313 (HEAL 19), and *Lactobacillus plantarum* DSM 15316 (HEAL 99) were deposited on 27 November 2002 at DSMZ-DEUTSCHE SAMMLUNG VON MIKROORGANISMEN UND ZELLKULTUREN GmbH, Mascheroder Weg 1b, D-38124 Braunschweig, Germany, by Probi AB.

*Lactobacillus plantarum* DSM 32131 (GOS42) was deposited on 2 September 2015 at Leibniz Institute DSMZ-German Collection of Microorganisms and Cell Cultures, Inhoffenstr. 7 B, D-38124 Braunschweig, Germany by Probi AB.

*Lactobacillus plantarum* DSM 17852 (LB3e) and *Lactobacillus plantarum* DSM 17853 (LB7c) were deposited on 6 January 2006 at DSMZ-DEUTSCHE SAMMLUNG VON MIKROORGANISMEN UND ZELLKULTUREN GmbH, Mascheroder Weg 1b, D-38124 Braunschweig, Germany, by Probac AB. All rights and duties in connection with microorganism deposits DSM 17852 and DSM 17853 were subsequently given to and accepted by Probi AB, who is now the depositor of the DSM 17852 and DSM 17853 strains.

Preferably, the at least one strain of *Lactobacillus plantarum* is *Lactobacillus plantarum* DSM 15312 (HEAL 9) and/or *Lactobacillus plantarum* DSM 15313 (HEAL 19).

According to the invention, the combination of live *Lactobacillus* strains is *Lactobacillus paracasei* DSM 13434 (8700:2) and at least one strain of *Lactobacillus plantarum.* For example, *Lactobacillus paracasei* 8700:2 (DSM 13434) may be used in combination with one, two or more probiotic strains of *Lactobacillus plantarum.*

In a most preferred embodiment according to the invention, the combination of *Lactobacillus* strains is *Lactobacillus paracasei* DSM 13434 (8700:2) in combination with *Lactobacillus plantarum* DSM 15312 (HEAL 9) and *Lactobacillus plantarum* DSM 15313 (HEAL 19).

Preferably, the live or probiotic strains are viable. For example, preferably the live or probiotic strains are freeze-dried.

### Compositions

The combination for use according to the invention may be present in a composition comprising at least one suitable carrier, i.e. one or more carriers.

For example, the carrier may be a diluent or excipient. The composition may be as a solid or liquid formulation, and hence the at least one carrier may be a solid or a liquid, or may comprise both at least one solid component and at least one liquid component.

Examples of a suitable liquid carrier include water, milk, coconut water, fruit drinks and juices, milk substitutes (soya drink, oat drink, nut and other plant-based drinks), sparkling beverages, glycerin, propylene glycol and other aqueous solvents.

Examples of a suitable solid carrier or excipient include maltodextrin, inulin, a cellulose such as microcrystalline cellulose (MCC), hydroxypropylmethylcellulose (HPMC) or hydroxy-propylcellulose (HPC), sugar alcohols, high molecular weight polyethylene glycols, lactose, sodium citrate, calcium carbonate, dibasic calcium phosphate and glycine, disintegrants such as starch (preferably corn, potato, tapioca or other vegetable starch), sodium starch glycollate, croscarmellose sodium and certain complex silicates, and granulation binders such as polyvinylpyrrolidone, sucrose, gelatin and acacia. Additionally, lubricating agents such as magnesium stearate, stearic acid, glyceryl behenate and talc may be included.

In an embodiment according to the invention, the carrier may be selected from a pharmaceutically acceptable carrier, a pharmaceutically acceptable excipient, a food-grade carrier, a food-grade excipient, a diluent and a food.

Examples of suitable pharmaceutically acceptable carriers, excipients and diluents include those well known to a skilled person in the art, for example those given in Remington: The Science and Practice of Pharmacy, 19th ed., vol. 1 & 2 (ed. Gennaro, 1995, Mack Publishing Company).

By "food-grade" we include carriers, ingredients and excipients that meet the `generally recognized as safe' (GRAS) criteria.

By "food" we include any substance for consumption to provide nutritional benefit or support for a mammal, preferably a human. Examples of suitable food carriers include beverages (e.g. juices), dairy products (e.g. yoghurts, cheese, ice creams, infant formula and spreads such as margarine), dairy-alternative products (e.g. soy, nut or other plant-based drinks, yoghurts and spreads), cereal-based products (e.g. breads, biscuits, breakfast cereals, pasta and dry food bars such as health bars), and baby food (e.g. pureed fruit and/or vegetable).

The composition according to embodiments of the invention may be a dry, non-fermented composition, a fermented composition, or a dry, fermented composition. Fermentation in this context particularly includes lactic acid fermentation by lactic acid bacteria in anaerobic conditions. In the case of a dry, non-fermented composition, substantially no fermentation takes place before ingestion by a subject, and so fermentation only takes place in the gastrointestinal tract after ingestion of the composition by a subject.

Hence, in some embodiments according to the invention, the composition in the form of a food wherein the food is a cereal-based product, a dairy product, a juice drink, or a fermented food.

Examples of fermented foods include fermented milk products (such as yoghurt, kefir or lassi), fermented dairy-free milk alternatives (such as coconut milk kefir), fermented cereal-based products (such as oats, oatmeal, maize, sorghum, wheat), fermented vegetables (such as sauerkraut, kimchi, or pickles), fermented legumes or soybeans (such as natto or tempeh) and fermented tea (such as kombucha).

In some embodiments according to the invention, the combination for use is present in a composition that is not naturally occurring, e.g. the composition comprises more than the live *Lactobacillus* strain(s) and water.

In use, the combination or the composition comprising the combination according to the invention may be mixed with a liquid or solid carrier before administration to a mammal. For example, a subject may mix the combination or the composition thereof with a carrier comprising one or more liquids chosen from water, milk, coconut water, fruit drinks and juices, milk substitutes (soya drink, oat drink, nut and other plant-based drinks), sparkling beverages or some other aqueous solvent or drink prior to intake. Similarly, the combination or the composition thereof may be mixed with a carrier consisting of one or more foods. Suitable food carriers include oatmeal carrier, barley carrier, fermented or non-fermented dairy products such as yoghurts, ice creams, milkshakes, fruit juices, beverages, soups, breads, biscuits, pasta, breakfast cereals, dry food bars including health bars, plant-based foods such as soy products, spreads, baby food, infant nutrition, infant formula, breast milk replacements from birth.

Preferably, the formulation is a unit dosage containing a daily dose or unit, daily sub-dose or an appropriate fraction thereof, of the composition comprising the live *Lactobacillus* strains.

The composition may be a dietary supplement. By "dietary supplement" we include the meaning of a manufactured product intended to supplement the diet when taken by mouth, e.g. as a pill, capsule, tablet, or liquid. Dietary supplements may contain substances that are essential to life and/or those that have not been confirmed as being essential to life but may have a beneficial biological effect. When the composition is in the form of a dietary supplement the carrier(s) to be added include those well known to a skilled person in the art, for example those given in Remington: The Science and Practice of Pharmacy, 19th ed., vol. 1 & 2 (ed. Gennaro, 1995, Mack Publishing Company), or any suitable food-grade carrier. Any other ingredients that are normally used in dietary supplements are known to a skilled person and may also be added conventionally together with the combination of live *Lactobacillus* strains.

The composition may be provided in the form of a solution, suspension, emulsion, tablet, granule, powder, capsule, lozenge, chewing gum, or suppository.

For example, in a preferred embodiment, the composition is a dietary supplement in the form of a capsule comprising freeze-dried *Lactobacillus,* such as a dietary supplement in the form of a capsule comprising 10¹⁰ CFU freeze-dried *Lactobacillus.*

In an embodiment according to the invention, the combination of live *Lactobacillus* strains is present (e.g. in a composition) in an amount from about 1×10⁶ to about 1×10¹⁴ CFU/dose, preferably from about 1×10⁸ to about 1×10¹² CFU/dose, more preferably from about 1×10⁹ to about 1×10¹¹ CFU/dose, and most preferably about 1×10¹⁰ CFU/dose, wherein such amounts represent the total CFU/dose of the combination of live *Lactobacillus* strains. For example, the combination may be present in an amount from about 1×10⁶, 1×10⁷, 1×10⁸, 1×10⁹, 1×10¹⁰, 1×10¹¹, 1×10¹² or about 1×10¹³ CFU/dose. The combination may be present in an amount to about 1×10¹⁴, 1×10¹³, 1×10¹², 1×10¹¹, 1×10¹⁰, 1×10⁹, 1×10⁸ or about 1×10⁷ CFU/dose. The combination for use according to the invention may also be used alone in water or any other aqueous vehicle in which the combination is added or mixed before ingestion.

In an embodiment according to the invention the combination for use is present in a composition that is supplemented with a chondroprotector. A chondroprotector (or chondroprotective compound) includes any specific compound or chemical that delays the progressive joint space narrowing characteristic of arthritis (or osteoarthritis). For example, a chondroprotector may perform one or more of: (1) stimulating chondrocyte synthesis of collagen and proteoglycans; (2) enhancing synoviocyte production of haluronan; (3) inhibiting cartilage degradation; (4) preventing fibrin formation in the vasculature. Any chondroprotector that is known to a skilled person may be added to the composition. In a preferred embodiment, the chondroprotector is selected from chondroitin sulphate, glucosamine and/or hyaluronic acid.

The composition can be administered orally, buccally or sublingually in the form of tablets, capsules, powders, ovules, elixirs, solutions or suspensions, which may contain flavouring or colouring agents, for immediate-, delayed- or controlled-release applications. The composition may be administered in the form of a powdered composition such as a fast-melt microbial composition, for example those described in WO 2017/060477 or in Probi's publication WO 2018/224509 relating to Probi^{®} Fast Melt technology. Where the powder is not in a fast-melt microbial composition, it may be suitable for being added to a food (e.g. yoghurt) or drink (e.g. water or milk) before ingestion.

Where the composition is in the form of a powder, it would typically be filled in a sealed container, which provides an oxygen and moisture barrier in order to protect and maintain the viability of the probiotic bacteria in the composition. Hence, where the composition is in the form of a powder, preferably the composition is packaged in sealed aluminium foil sticks, where each stick comprises one dose of the composition, *i.e.* one dose of the probiotic bacteria. Non-limiting examples of suitable containers include a stick, bag, pouch or capsule. In a preferred embodiment, the container is an aluminium foil or a polyethylene stick, which is typically sealed by welding. The stick is typically configured for easy tear opening. The stick may have a tear notch.

The composition comprising the combination for use according to the invention may be formulated as a controlled-release solid dosage form, for example any of those described in WO 03/026687 and US Patent Nos. 8,007,777 and 8,540,980. The composition may be formulated as a layered dosage form, for example Probi's BIO-*tract*^{®} technology including any of the layered dosage forms described in WO 2016/003870.

A tablet may be made by compression or moulding, optionally with one or more accessory ingredients. Compressed tablets may be prepared by compressing in a suitable machine the probiotic strains (e.g. freeze-dried) in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide the desired release profile.

### Pharmaceutical compositions

In accordance with the claims, the combination for use according to the invention may be present in a pharmaceutical composition comprising one or more pharmaceutically acceptable excipients. Preferably the mammal is a non-rodent mammal, more preferably a human, most preferably a man or a woman older than 45, 50, 55, 60, 65, 70, 75 or 80. For example, the preferences for the subjects (e.g. patient group) for which the combination for use present in a pharmaceutical composition may be suitable includes those preferences given above in the section entitled Patient Group.

Such a pharmaceutical composition may be a composition as described above in the section entitled Compositions. The term "pharmaceutically acceptable" includes that the one or more excipients must not be deleterious to the recipients thereof and must be compatible with the combination for use according to the invention. Examples of such pharmaceutically acceptable excipients are well known in the art and include those described above in the section entitled Compositions, for example those described in Remington: The Science and Practice of Pharmacy, 19th ed., vol. 1 & 2 (ed. Gennaro, 1995, Mack Publishing Company).

For example, the pharmaceutical composition may be formulated as a controlled-release solid dosage form, e.g. any of those described in WO 03/026687 and US Patent Nos. 8,007,777 and 8,540,980, or the pharmaceutical composition may be formulated as a layered dosage form, e.g. any of those described in WO 2016/003870.

The one or more pharmaceutically acceptable excipients may be water or saline which will be sterile and pyrogen free.

Preferably, the combination for use present in a pharmaceutical composition may be administered by any conventional method including oral and tube feeding. Administration may consist of a single dose or a plurality of doses over a period of time.

### Methods of treatment

References herein to methods of treatment are to be interpreted as references to a strain, combination of strains or composition for use in those methods, such as the subject matter set forth in the claims.

Otherwise disclosed herein are methods for treating and/or preventing osteoarthritis in a mammal, comprising administering to a mammal in need thereof an effective dose of the at least one probiotic strain disclosed herein, such as when present in a composition as disclosed herein and/or a pharmaceutical composition as disclosed herein. Preferably, the methods include those wherein the treatment and/or prevention of osteoarthritis is by treating and/or preventing one or more of cartilage damage, bone damage at a joint, and inflammation associated with osteoarthritis. Preferably, the treatment and/or prevention of osteoarthritis is by treating and/or preventing cartilage damage. Preferably, the treatment and/or prevention of osteoarthritis is by treating and/or preventing bone damage at a joint. Preferably, the treatment and/or prevention of osteoarthritis is by treating and/or preventing inflammation associated with osteoarthritis, preferably by treating and/or preventing inflammation localised to a joint.

The mammal on which the methods disclosed herein are carried out (including in accordance with the claimed invention) may be any mammal given above in the section entitled Patient Group. Preferably, the mammal is male, particularly a man. The mammal may be female, particularly a woman, such as a peri-menopausal woman or a post-menopausal woman. For example, the woman may be within one, two, three, four, five, six, seven, eight, nine, ten, eleven or twelve years from onset of menopause, such as a woman six years or less after onset of menopause. Hence, in one embodiment of the methods disclosed herein, treatment commences six years or less after onset of menopause.

Administration according to the methods disclosed herein may include administration orally, buccally or sublingually as described above.

Administration according to the methods disclosed herein may include administration at least every one, two, three, four, five, six or seven days, or at least one, two, three, four, five, six or seven times a week. Preferably administration takes place once daily.

Administration according to the methods disclosed herein may include administration that is repeated for up to one, two, three, four, five or six days, for up to one, two, three, four or five weeks, for up to one, two, three, four, five, six, seven, eight, nine, ten, eleven or twelve months, or for more than one, two or three years or longer. Preferably, administration is repeated for at least 7 days, such as for at least one week, two weeks, three weeks, more preferably for at least four weeks, one month, two months or three months, and even more preferably for at least six months, nine months or one year.

Administration according to the methods disclosed herein is preferably of a unit dosage of from about 1×10⁶ to about 1×10¹⁴ CFU/unit dose, preferably from about 1×10⁸ to about 1×10¹² CFU/unit dose, more preferably from about 1×10⁹ to about 1×10¹¹ CFU/unit dose, and most preferably about 1×10¹⁰ CFU/unit dose, as described above. Administration according to the methods disclosed herein preferably results in an effective dose of from about 1×10⁶ to about 1×10¹⁴ CFU/unit dose, preferably from about 1×10⁸ to about 1×10¹² CFU/unit dose, more preferably from about 1×10⁹ to about 1×10¹¹ CFU/unit dose, and most preferably about 1×10¹⁰ CFU/unit dose. Preferably, each subject is administered one unit dose per day. Hence, administration according to the methods disclosed herein preferably results in a daily dose of from about 1×10⁶ to about 1×10¹⁴ CFU/day, preferably from about 1×10⁸ to about 1×10¹² CFU/day, more preferably from about 1×10⁹ to about 1×10¹¹ CFU/day, and most preferably about 1×10¹⁰ CFU/day.

It will be appreciated that a preferable daily dose may also be achieved by administration of more than one sub-dose, for example, by a twice daily administration of a unit dose comprising half of the preferable daily dose. Hence, the preferred ranges for the effective dose may also represent the preferred daily dosage to be achieved in whatever number of unit doses is practical.

The subject may be instructed to consume a therapeutically effective amount of the combination for use according to the invention (optionally when present in a composition or a pharmaceutical composition), in combination with water, another aqueous solvent or a food product, e.g. yoghurt.

The listing or discussion of an apparently prior-published document in this specification should not necessarily be taken as an acknowledgement that the document is part of the state of the art or is common general knowledge.

The invention will now be described in more detail by reference to the following Examples and Figures.

### Brief description of the figures

Fig. **1** discloses the project plan diagram for Experimental Example 1.

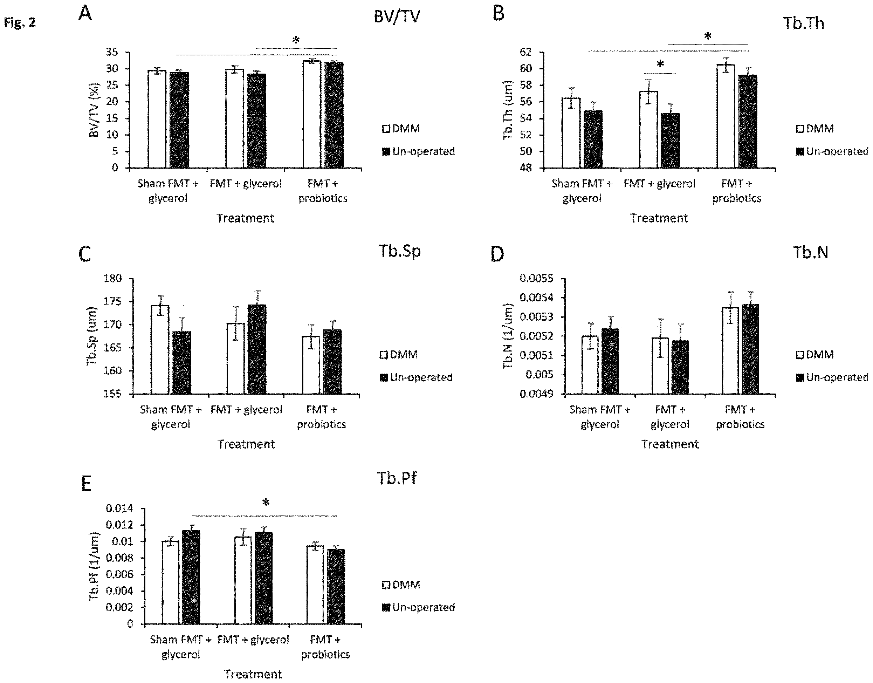

Fig. **2** discloses the effect of probiotics on the tibial epiphyseal trabecular bone (trabecular bone at the proximal epiphysis of the tibia). (A) Trabecular bone volume/total volume (BV/TV) of DMM-operated and unoperated knee joints of male mice, as assessed by microCT of the tibial epiphysis. (B) Trabecular thickness (Tb.Th); (C) Trabecular separation (Tb.Sp); (D) Trabecular number (Tb.N); and (E) Trabecular pattern factor (Tb.Pf), from the same experiment. Values are mean ± SEM from 11-13 mice per group. Significant differences between groups indicated by * = p <0.05.

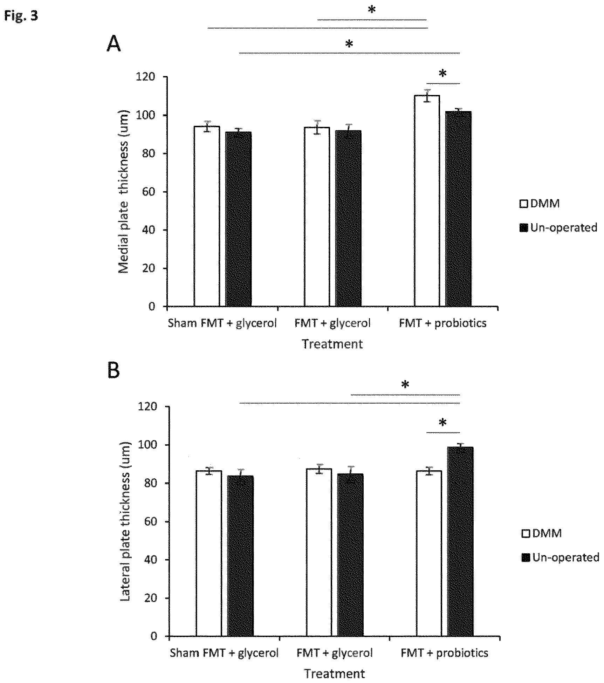

Fig. **3** discloses the effect of probiotics on the tibial subchondral bone plates (medial and lateral subchondral bone plates), which comprise cortical bone. (A) Medial plate thickness of DMM-operated and unoperated knee joints of male mice, as assessed by microCT of the tibial subchondral bone. (B) Lateral plate thickness from the same experiment. Values are mean ± SEM from 11-13 mice per group. Significant differences between groups indicated by * = p <0.05.

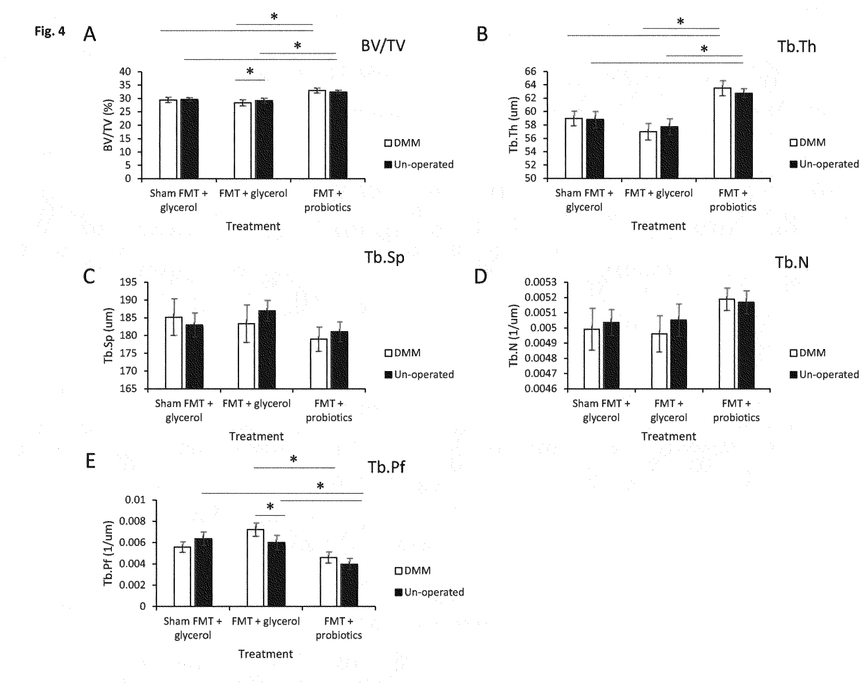

Fig. **4** discloses the effect of probiotics on the femoral epiphyseal trabecular bone (trabecular bone at the distal epiphysis of the femur). (A) Trabecular bone volume/total volume (BV/TV) of DMM-operated and unoperated knee joints of male mice, as assessed by microCT of the femoral epiphysis. (B) Trabecular thickness (Tb.Th); (C) Trabecular separation (Tb.Sp); (D) Trabecular number (Tb.N); and (E) Trabecular pattern factor (Tb.Pf), from the same experiment. Values are mean ± SEM from 10-13 mice per group. Significant differences between groups indicated by * = p <0.05.

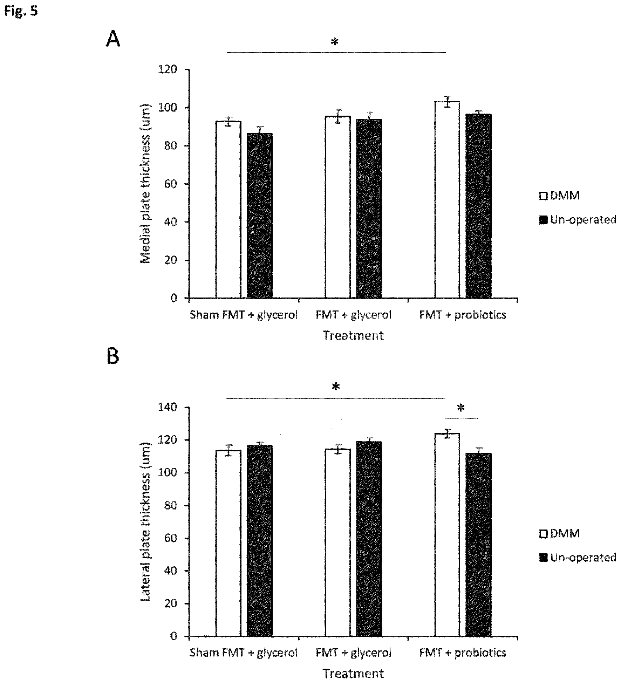

Fig. **5** discloses the effect of probiotics on the femoral subchondral bone plates (medial and lateral subchondral bone plates), which comprise cortical bone. (A) Medial plate thickness of DMM-operated and unoperated knee joints of male mice, as assessed by microCT of the femoral subchondral bone. (B) Lateral plate thickness from the same experiment. Values are mean ± SEM from 10-13 mice per group. Significant differences between groups indicated by * = p <0.05.

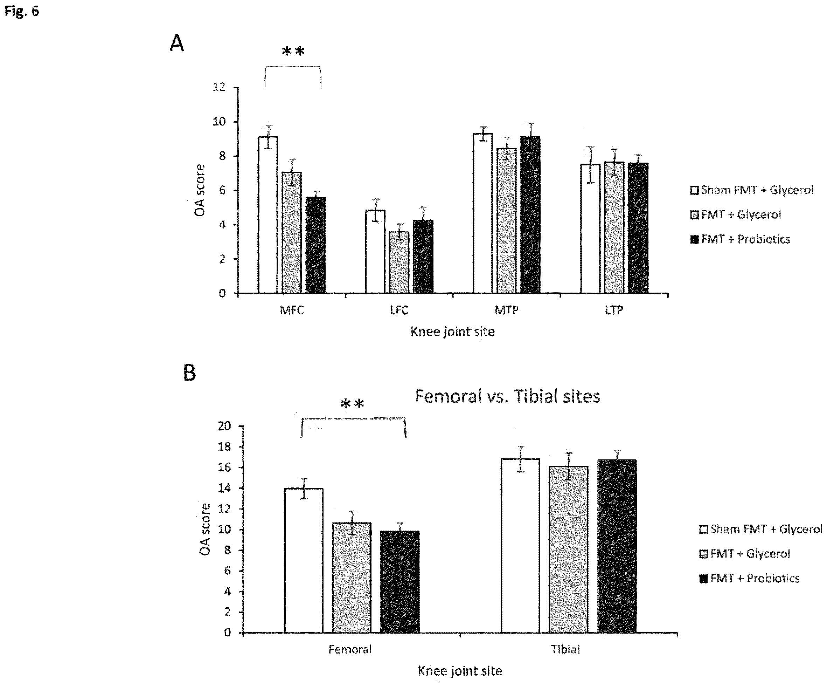

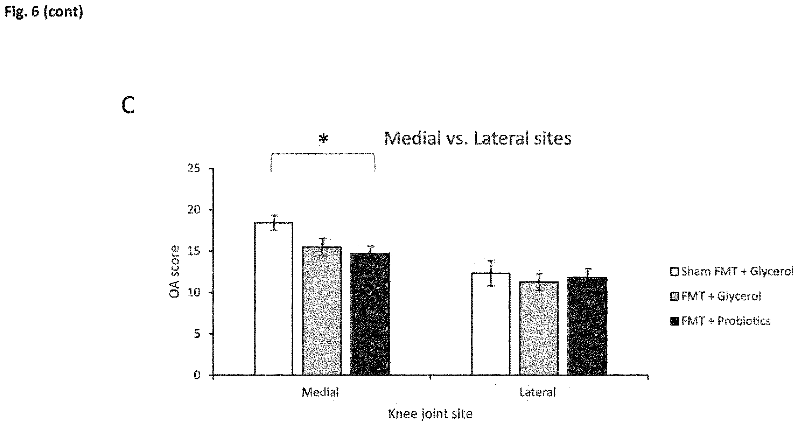

Fig. **6** discloses the effect of probiotics on DMM-induced cartilage damage. (A) Osteoarthritis (OA) score at four sites - medial femoral condyle (MFC), lateral femoral condyle (LFC), medial tibial plateau (MTP) and lateral tibial plateau (LTP) - in DMM-operated knee joints of male mice. (B) Sum of OA score at femoral sites vs. tibial sites from the same experiment. (C) Sum of OA score at medial vs. lateral sites from the same experiment. Values are mean ± SEM from 10-13 mice per group. Significant differences between groups are indicated by * = *p* < 0.05 and ** = *p <* 0.01.

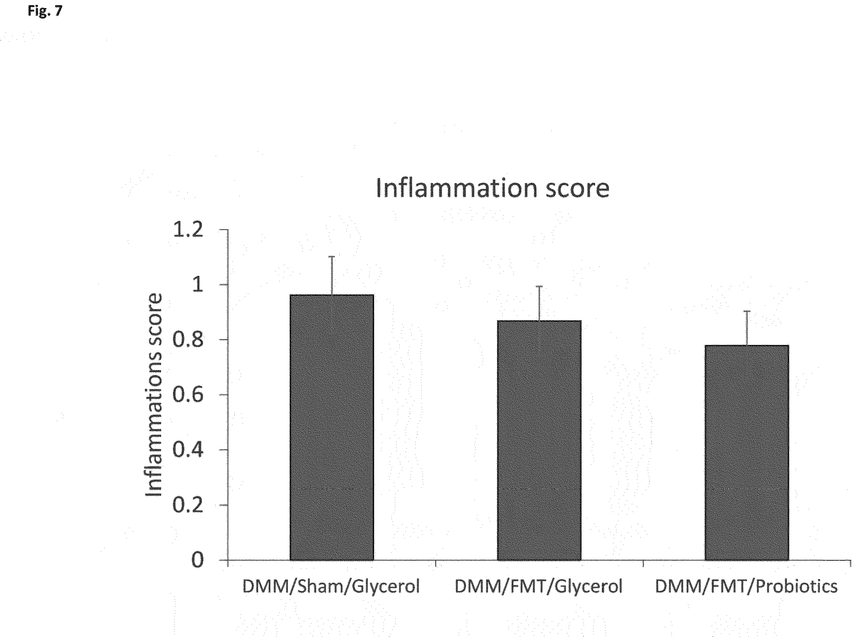

Fig. **7** discloses the effect of probiotics on inflammation in DMM knee joint. Inflammation is scored according to the semi-quantitative synovitis scoring system of Jackson *et al,* 2014 (see Table 2, *infra*)*.* Values are mean ± SEM from 10-13 mice per group.

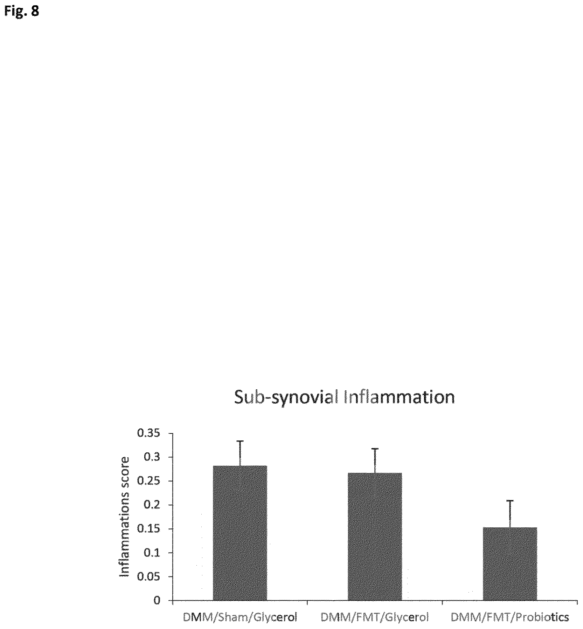

Fig. **8** discloses the effect of microbiome and probiotics on the specific inflammation measure of sub-synovial inflammation in the DMM knee joint, scored according to the semi-quantitative synovitis scoring system of Jackson *et al,* 2014 (see Table 2, *infra*)*.* Values are mean ± SEM from 10-13 mice per group.

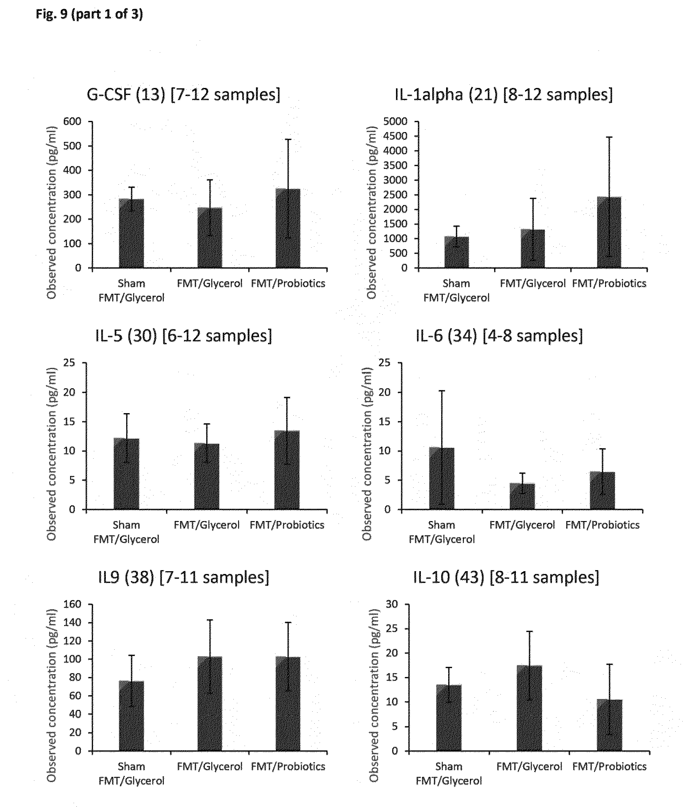

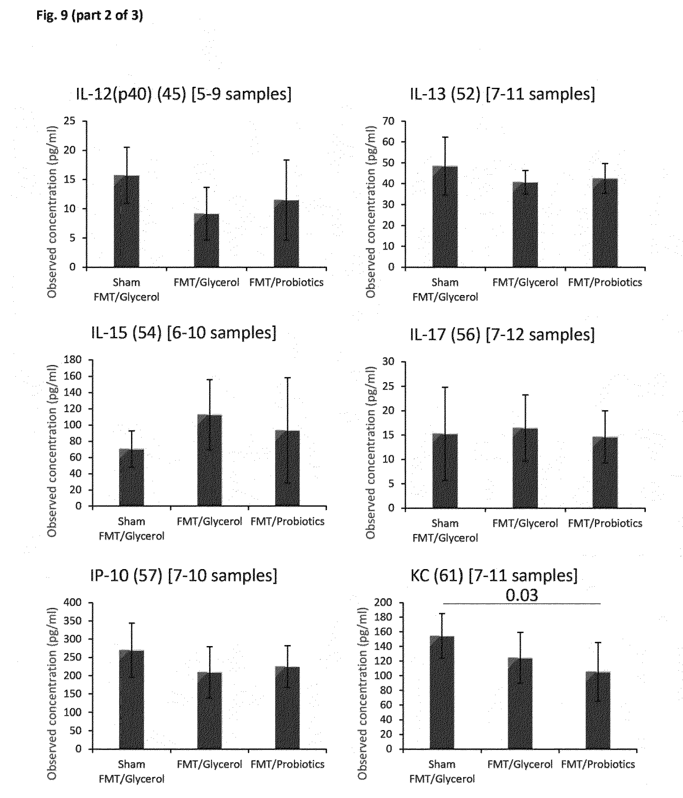

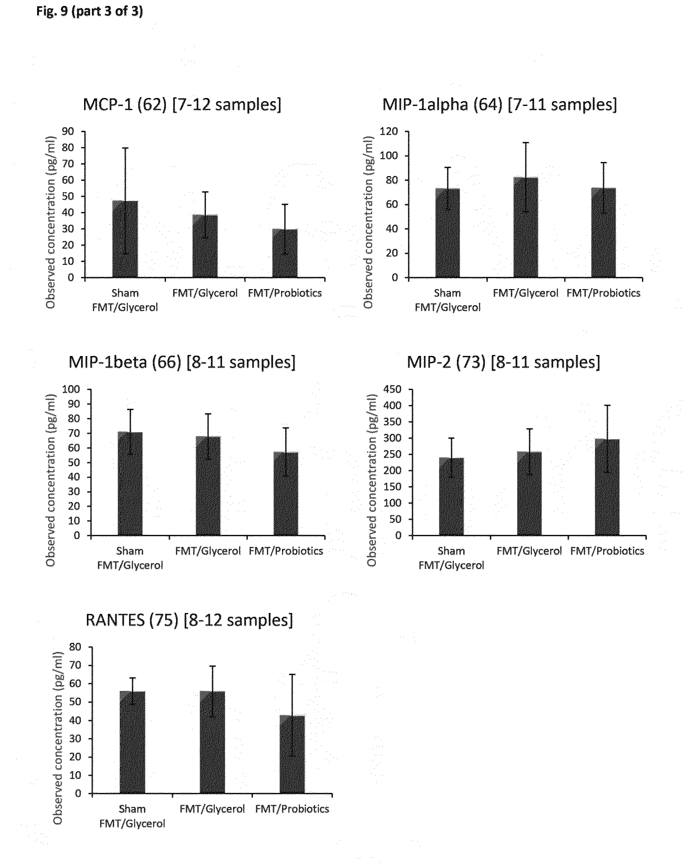

Fig. **9** discloses the effect of microbiome and probiotics on the concentration of inflammatory cytokines in mice with DMM-induced cartilage damage. Mouse serum samples from the microbiome experiment were analysed by MILLIPLEX^{®} MAP Mouse Cytokine/Chemokine Magnetic Bead Panel. In total 17 mouse cytokines were investigated. Graphs show the observed concentration of each cytokine across the three different interventions (pg/mL). Values are mean ± SD from 4-12 mice per group (actual sample number per cytokine is indicated in the title of each graph). Outliers were identified using the `Interquartile range rule for outliers'. Significant p values are shown between pairs of samples.

### Exemplary dosage forms

In addition to the formulations referenced above, the following examples illustrate pharmaceutical formulations according to the invention.

### Example A: Tablet

| | |
|---|---|
| Probiotic strain(s) | 1x10¹⁰ CFU |
| Lactose | 200 mg |
| Starch | 50 mg |
| Polyvinylpyrrolidone | 5 mg |
| Magnesium stearate | 4 mg |

Tablets are prepared from the foregoing ingredients by wet granulation followed by compression.

### Example B: Tablet Formulations

The following formulations A and B are prepared by wet granulation of the ingredients with a solution of povidone, followed by addition of magnesium stearate and compression.

### Formulation A

| | | |
|---|---|---|
| (a) Probiotic strain(s) | 1x10¹⁰ CFU | 1x10¹⁰ CFU |
| (b) Lactose B.P. | 210 mg | 26 mg |
| (c) Povidone B.P. | 15 mg | 9 mg |
| (d) Sodium Starch Glycolate | 20 mg | 12 mg |
| (e) Magnesium Stearate | 5 mg | 3 mg |

### Formulation B

| | | |
|---|---|---|
| (a) Probiotic strain(s) | 1x10¹⁰ CFU | 1x10¹⁰ CFU |
| (b) Lactose | 150 mg | - |
| (c) Avicel PH 101^{®} | 60 mg | 26 mg |
| (d) Povidone B.P. | 15 mg | 9 mg |
| (e) Sodium Starch Glycolate | 20 mg | 12 mg |
| (f) Magnesium Stearate | 5 mg | 3 mg |

### Formulation C

| | |
|---|---|
| Probiotic strain(s) | 1x10¹⁰ CFU |
| Lactose | 200 mg |
| Starch | 50 mg |
| Povidone | 5 mg |
| Magnesium stearate | 4 mg |

The following formulations, D and E, are prepared by direct compression of the admixed ingredients. The lactose used in formulation E is of the direction compression type.

### Formulation D

| | |
|---|---|
| Probiotic strain(s) | 1x10¹⁰ CFU |
| Pregelatinised Starch NF15 | 150 mg |

### Formulation E

| | |
|---|---|
| Probiotic strain(s) | 1x10¹⁰ CFU |
| Lactose | 150 mg |
| Avicel ^{®} | 100 mg |

### Formulation F (Controlled Release Formulation

The formulation is prepared by wet granulation of the ingredients (below) with a solution of povidone followed by the addition of magnesium stearate and compression.

| | |
|---|---|
| (a) Probiotic strain(s) | 1x10¹⁰ CFU |
| (b) Hydroxypropylmethylcellulose | 112 mg |

### (Methocel K4M Premium)^{®}

| | |
|---|---|
| (c) Lactose B.P. | 53 mg |
| (d) Povidone B.P.C. | 28 mg |
| (e) Magnesium Stearate | 7 mg |

Release takes place over a period of about 6-8 hours and was complete after 12 hours.

### Example C: Capsule Formulations

### Formulation A

A capsule formulation is prepared by admixing the ingredients of Formulation D in Example B above and filling into a two-part hard gelatin capsule. Formulation B (*infra*) is prepared in a similar manner.

### Formulation B

| | |
|---|---|
| (a) Probiotic strain(s) | 1x10¹⁰ CFU |
| (b) Lactose B.P. | 143 mg |
| (c) Sodium Starch Glycolate | 25 mg |
| (d) Magnesium Stearate | 2 mg |

### Formulation C

| | |
|---|---|
| (a) Probiotic strain(s) | 1x10¹⁰ CFU |
| (b) Macrogol 4000 BP | 350 mg |

Capsules are prepared by melting the Macrogol 4000 BP, dispersing the probiotic strain(s) in the melt and filling the melt into a two-part hard gelatin capsule.

### Formulation D (Controlled Release Capsule)

The following controlled release capsule formulation is prepared by extruding ingredients a, b, and c using an extruder, followed by spheronisation of the extrudate and drying. The dried pellets are then coated with release-controlling membrane (d) and filled into a two-piece, hard gelatin capsule.

| | |
|---|---|
| (a) Probiotic strain(s) | 1×10¹⁰ CFU |
| (b) Microcrystalline Cellulose | 125 mg |
| (c) Lactose BP | 125 mg |
| (d) Ethyl Cellulose | 13 mg |

### Example D: Powder formulations

### Formulation A (fast-melting microbial composition)

| | |
|---|---|
| (a) Probiotic strain(s) | 80 mg (preferably 1×10¹⁰ CFU) |
| (b) Erythritol | 450 mg |
| (c) Inulin | 227.5 mg |
| (d) Xylitol | 227.5 mg |
| (e) Lemon flavour | 10 mg |
| (f) Silicon dioxide | 5 mg |

### Formulation B (fast-melting microbial composition)

| | |
|---|---|
| (a) Probiotic strain(s) | 80 mg (preferably 1×10¹⁰ CFU) |
| (b) Erythritol | 425 mg |
| (c) Inulin | 215 mg |
| (d) Xylitol | 215 mg |
| (e) Maltodextrin | 50 mg |
| (f) Lemon flavour | 10 mg |
| (g) Silicon dioxide | 5 mg |

### Experimental example 1 - effect of probiotics on osteoarthritis (OA) development over and above normal microbiome in DMM-operated mice.

We examined the role of the intestinal microbiome in the development of osteoarthritis in mice subjected to destabilisation of medial meniscus (DMM) and assessed the effect of probiotics on osteoarthritis development over and above normal microbiome.

Regarding the choice of model for this study, we considered the findings of Glasson et al (2007, The surgical destabilization of the medial meniscus (DMM) model of osteoarthritis in the 129/SvEv mouse, Osteoarthr Cartil 15(9):1061-1069), who evaluated the anterior cruciate ligament transection (ACLT) and destabilisation of the medial meniscus (DMM) surgical instability models of osteoarthritis. They reported that ACLT causes a more severe damage whereas DMM causes a mild-to-moderate damage, which resembles spontaneous OA development.

### Materials and methods

Antibiotic treatment was used to deplete mouse intestinal microbes as previously shown (Ellekilde et al, 2014, Sci Rep 4:5922; Reikvam et al, 2011, PLoS One 6(3):e17996). Initially, ampicillin was administered to pregnant mice in drinking water *ad libitum* from one week before birth until the progeny mice reach the age of 3 weeks (i.e. age of weaning). In addition to ampicillin in drinking water, an antibiotic cocktail consisting of vancomycin, neomycin, metronidazole and amphotericin-B ampicillin was administered to the progeny mice daily by gavage as previously described (Reikvam et al, 2011, PLoS One 6(3):e17996) for 3 weeks after weaning. At the end of that period (six weeks after birth) faecal samples from healthy mice were aseptically removed and transplanted to the antibiotic-treated hosts (faecal microbiota transplantation, FMT), as previously described (Ellekilde et al, 2014, Sci Rep 4:5922). Briefly, samples were diluted 1:10 in a 50% glycerol/PBS solution, frozen in liquid nitrogen and kept in -80°C. At the day of the inoculation, the faecal solution was further diluted in 1:5 and then administered via oral gavage (0.15ml per recipient mouse). This enabled the gut microbiome to be restored. Sham reconstitution of the gut microbiome involved oral gavage of glycerol/PBS solution.

All mice were subjected to destabilisation of the medial meniscus (DMM) at the age of 8 weeks old. DMM was performed on one leg using an operating microscope. The contralateral knee joint was left un-operated and was used as an internal control to evaluate the effect of DMM on the development of OA. Probiotic or vehicle (glycerol) treatment began two weeks before DMM (i.e. at 6 weeks of age) and continued for 8 weeks following surgery (i.e. until 16 weeks of age) in order to study the preventive effect of probiotic treatment on DMM-induced OA. Probiotic treatment was a mixture of three *Lactobacillus* (L) strains, *L. paracasei* DSM 13434, *L. plantarum* DSM 15312 and DSM 15313. *Lactobacillus* strains were administered in drinking water according to instructions provided by Probi AB (10 mL of study product, containing equal amounts of each one of the three bacterial strains, was diluted with water to 600 mL in order to have a final total concentration of 10⁹ CFU/mL). Control groups received water with vehicle (glycerol) (see Figure 1 for project plan diagram). Eight weeks following surgery (i.e. at 16 weeks of age), mice were sacrificed by CO₂ asphyxiation and knee joints were scanned by microCT to look for quantitative and qualitative changes in subchondral bone.

MicroCT analysis of knee joints was performed as described in van 't Hof (2012, Analysis of bone architecture in rodents using microcomputed tomography, Methods Mol Biol 816:461-476), Bouxsein et al (2010, Guidelines for assessment of bone microstructure in rodents using micro-computed tomography, J Bone Miner Res 25:1468-1486) and Campbell and Sophocleous (2014, Quantitative analysis of bone structure by micro-computed tomography, BoneKEy Reports, 3:564). Analysis of periarticular bone was performed by microCT as previously described in Sophocleous et al (2015, The type 2 cannabinoid receptor regulates susceptibility to osteoarthritis in mice, Osteoarthr Cartil 23:1585-1594). Briefly, a Skyscan 1172 instrument was set at 60 kV and 167mA. Tibial and femoral epiphyseal trabecular bone analysis was performed in the coronal plane, at a resolution of 5mm. Following acquisition, the images were reconstructed using the Skyscan NRecon programme and analysed using Skyscan CTAn software.

Bone mineral density (BMD) was not measured in this animal study, but trabecular bone volume (BV/TV) was measured by microCT instead.

Histology analysis of knee joint was also performed as detailed in Glasson et al (2007, The surgical destabilization of the medial meniscus (DMM) model of osteoarthritis in the 129/SvEv mouse, Osteoarthr Cartil 15(9):1061-1069), Glasson SS et al (2010, The OARSI histopathology initiative - recommendations for histological assessments of osteoarthritis in the mouse, Osteoarthr Cartil 18:S17-23) and Sophocleous and Huesa (2019, Osteoarthritis Mouse Model of Destabilization of the Medial Meniscus, pp 281-293 in Idris [ed], Bone Research Protocols, Methods in Molecular Biology, vol 1914, Humana Press, New York, NY). In particular, histological analysis focused on cartilage and an assessment of localised inflammation at the knee joint. Histological analysis was performed as previously described in Sophocleous *et al* (2015, *supra*)*.* Briefly, fixed whole joints were decalcified in 10% formic acid for 1 week. The joints were then processed and embedded in paraffin wax according to standard techniques. Coronal sections of 7µm thickness were taken through the entire joint at 45µm intervals. Sections were then stained with Safranin-O and Haematoxylin. Histological evaluation of the severity of osteoarthritis was performed by an observer blinded to pharmacological treatment according to the Osteoarthritis Research Society International (OARSI) semi-quantitative scoring system of Glasson *et al,* 2010, *supra*, reproduced in Table 1. Scores were generated separately from the medial tibial plateau (MTP), the medial femoral condyle (MFC), the lateral tibial plateau (LTP) and lateral femoral condyle (LFC).

**Table 1 - Semi-quantitative OARSI scoring system (Glasson et al, 2010, supra)**

| Grade | Osteoarthritic damage |
|---|---|
| 0 | Normal |
| 0.5 | Loss of Safranin-O without structural changes |
| 1 | Small fibrillations without loss of cartilage |
| 2 | Vertical clefts down to the layer immediately below the superficial layer and some loss of surface lamina |
| 3 | Vertical clefts/erosion to the calcified cartilage extending to <25% of the articular surface |
| 4 | Vertical clefts/erosion to the calcified cartilage extending to 25-50% of the articular surface |
| 5 | Vertical clefts/erosion to the calcified cartilage extending to 50-75% of the articular surface |
| 6 | Vertical clefts/erosion to the calcified cartilage extending to >75% of the articular surface |

Assessment of localised inflammation of the knee joint was carried out as described in Jackson et al (2014, Depletion of protease-activated receptor 2 but not protease-activated receptor 1 may confer protection against osteoarthritis in mice through extracartilaginous mechanisms, Arthritis Rheumatol66(12):3337-3348). In particular, the system for scoring localised inflammation was according to the semi-quantitative synovitis scoring system of Jackson *et al,* 2014, *supra*, reproduced in Table 2 below:

**Table 2 - Semi-quantitative synovitis scoring system (Jackson et al, 2014, supra)**

| **Score** | **Pannus** | **Synovial Hyperplasia Severity** | **Sub-synovial Inflammation** |
|---|---|---|---|
| **Description** | Defined as fibrous tissue/synovium/ inflammatory cell outgrowth spreading over the surface of the bone and/or cartilage at the joint margins. | Score this superior to the meniscal remnant. Do not score the cells actually attached to the tibia or femur or cells on the surface of the meniscus itself and avoid peri-meniscal plica. Score the maximum hyperplasia seen anywhere along this area. | Score this superior to the meniscal remnant. The infiltration of inflammatory cells (neutrophils, macrophages and/or lymphocytes) is evaluated. |
| **0** | No Pannus | 1 cell thick | No inflammatory cells |
| **1** | Mild: Pannus has migrated on bone but not encroaching on cartilage. | Mild: 2-3 cells thick | Occasional scattered inflammatory cells - or perivascular. |
| **2** | Moderate: Pannus has migrated < 1x cartilage depth. | Moderate: 4-5 cells thick | Focal areas of dense subsynovial white blood cell (WBC) infiltrate - but still predominantly normal subsynovial areolar connective tissue present. |
| **3** | Severe: Pannus has migrated > 1x cartilage depth. | Severe: > 6 cells thick | Widespread dense subsynovial WBC infiltrate - markedly reduced or little/no normal areolar connective tissue evident or lymphoid follicle formation. |

All analyses were performed on the following three groups of male mice:
**Group 1:** "Sham FMT + glycerol" (mice that had no faecal microbiota transplantation and only administration of vehicle [glycerol]);
**Group 2:** "FMT + glycerol" (mice that had faecal microbiota transplantation followed by administration of vehicle [glycerol]);
**Group 3:** "FMT + probiotics" (mice that had faecal microbiota transplantation followed by administration of probiotics).

Male mice were selected because OA severity has been shown to be markedly higher in males than females after DMM (Ma et al, 2007, Osteoarthritis severity is sex dependent in a surgical mouse model, Osteoarthritis Cartilage, 15(6):695-700).

### Statistical methods

Significant differences between groups were assessed using one-way analysis of variance (ANOVA) followed by Tukey HSD post hoc test (for equal variances) or Games- Howell post hoc test (for unequal variances). The significance level was set at p < 0.05.

### Results

### Increased measures of tibial epiphyseal trabecular bone at the knee joint with probiotic administration in unoperated knees

Fig. 2 shows that reconstituting the microbiome with FMT did not have a significant impact on any bone indices measured in this study.

Fig. 2A shows that tibial epiphyseal trabecular bone volume (as a proportion of total volume) of the unoperated knee was increased in the "FMT + probiotics" group compared to both the "FMT + glycerol" and "Sham FMT + glycerol" groups. No statistically significant difference in tibial epiphyseal trabecular bone volume of the operated knee was observed between groups.

Likewise, Figure 2B shows that tibial epiphyseal trabecular thickness of the unoperated knee was increased in the "FMT + probiotics" group compared to both the "FMT + glycerol" and "Sham FMT + glycerol" groups. No statistically significant difference in tibial epiphyseal trabecular thickness of the operated knee was observed between groups. However, in the "FMT + glycerol" group only, the tibial epiphyseal trabecular thickness was significantly higher in the operated vs unoperated knee.

Fig. 2C and Fig. 2D show that tibial epiphyseal trabecular separation and tibial epiphyseal trabecular number, respectively, were not significantly different between the groups.

However, Fig. 2E shows that tibial epiphyseal trabecular pattern factor of the unoperated knee was reduced in the "FMT + probiotics" group compared to the "Sham FMT + glycerol" group. Trabecular pattern factor (Tb.Pf) is related to trabecular connectivity. A higher Tb. Pf value indicates lower connectivity, while a lower Tb. Pf value indicates better (higher) connectivity amongst trabeculi. Hence, the results show that probiotic administration improved tibial epiphyseal trabecular connectivity. It appears likely that this result is due to increased trabecular thickness (Fig. 2B) and overall increased trabecular bone volume (Fig. 2A) rather than due to increased trabecular number (Fig. 2D). No statistically significant difference in tibial epiphyseal trabecular pattern factor of the operated knee was observed between groups.

### Increased measures of tibial subchondral bone plates at the knee joint with probiotic administration in unoperated knees

Fig. 3A shows that medial plate thickness of the tibial subchondral bone plate (cortical rather than trabecular bone) of both the operated and unoperated knees is increased in the "FMT + probiotics" group compared to the "Sham FMT + glycerol" group. Further, the medial plate thickness of the tibial subchondral bone plate of the operated knee in the "FMT + probiotics" group is increased compared to the unoperated knee of the "FMT + probiotics" group and also compared to the operated knee of the "FMT + glycerol" group.

Fig. 3B shows that lateral plate thickness of the tibial subchondral bone plate (cortical rather than trabecular bone) of the unoperated knee is increased in the "FMT + probiotics" group compared to the "FMT + glycerol" and "Sham FMT + glycerol" groups and compared to the operated knee of the "FTM + probiotics" group. Unlike for medial plate thickness, no increase in lateral plate thickness of the tibial subchondral bone plate is observed in the operated knee of the "FMT + probiotics" group compared to any other group.

Hence, the results for medial vs lateral bone plates are similar for the unoperated knee, but statistically significant results for the operated knee were only observed for the thickness of the medial plate.

### Increased measures of femoral epiphyseal trabecular bone at the knee joint with probiotic administration in operated and unoperated knees

Fig. 4 shows that reconstituting the microbiome with FMT did not have a significant impact on any bone indices at the epiphyseal bone of the femur.

However, administration of probiotics ("FMT + probiotics" group), increased femoral epiphyseal trabecular bone volume (as a proportion of total volume; BV/TV; Fig. 4A) and femoral epiphyseal trabecular thickness (Tb.Th.; Fig. 4B) in both the DMM-operated and un-operated knee joints compared to both the "FMT + glycerol" and "Sham FMT + glycerol" groups.

Fig. 4C and Fig. 4D show that femoral epiphyseal trabecular separation and femoral epiphyseal trabecular number, respectively, were not significantly different between the groups.

Fig. 4E shows that femoral epiphyseal trabecular pattern factor of the unoperated knee was reduced in the "FMT + probiotics" group compared to both the "FMT + glycerol" and "Sham FMT + glycerol" groups, indicating that probiotic administration improved femoral epiphyseal trabecular connectivity. Like for tibial epiphyseal trabecular connectivity, it appears likely that this result is due to increased trabecular thickness (Fig. 4B) and overall increased trabecular bone volume (Fig. 4A) rather than due to increased trabecular number (Fig. 4D).

Fig. 4E also shows that femoral epiphyseal trabecular pattern factor of the DMM-operated knee was reduced in the "FMT + probiotics" group compared to the "FMT + glycerol" group, indicating a better trabecular connectivity.

### Increased measures of femoral subchondral bone plates at the knee joint with probiotic administration in operated knees

Fig. 5A shows that medial plate thickness of the tibial subchondral bone plate (cortical rather than trabecular bone) of the operated knees is increased in the "FMT + probiotics" group compared to the "Sham FMT + glycerol" group.

Fig. 5B shows that lateral plate thickness of the tibial subchondral bone plate (cortical rather than trabecular bone) of the operated knee is increased in the "FMT + probiotics" group compared to the "Sham FMT + glycerol" group and compared to the unoperated knee of the "FTM + probiotics" group.

### Protective effects of probiotic administration on DMM-induced cartilage damage

Fig. 6A shows that osteoarthritis score (based on cartilage histology) assessed at the medial femoral condyle (MFC) of the operated knee was significantly reduced in the "FMT + probiotics" group compared to the "Sham FMT + glycerol" group (reduction of 38.8% ± 4.1%; *p* = 0.001). Fig. 6B and Fig. 6C further show that a reduction in osteoarthritis score was also present for the femoral sites as a whole (medial and lateral femoral condyles; reduction of 29.6% ± 5.7%; *p* = 0.008) and for the medial sites as a whole (medial femoral condyle and medial tibial plateau; reduction of 20.2% ± 4.9%; *p* = 0.019).

### Protective effects of probiotic administration on DMM-induced inflammation localised at the knee joint

Fig. 7 shows that the inflammation score assessed at the operated knee was reduced in the "FMT + probiotics" group (mean score = 0.78) compared to the "Sham + glycerol" group (mean score = 0.96) and "FMT + glycerol" group (mean score = 0.87).

### Protective effects of probiotic administration on DMM-induced sub-synovial inflammation at the knee joint

Fig. 8 shows that the specific modality of sub-synovial inflammation assessed at the operated knee (i.e. by the scoring system of Table 2, *infra* [Jackson *et al,* 2014]) was reduced in the "DMM/FMT/probiotics" group (mean score = 0.15) compared to the "DMM/Sham/glycerol" group (mean score = 0.28) and "DMM/FMT/glycerol" group (mean score = 0.27).

### Conclusions

The data in Fig. 2-5 show that administration of probiotic treatment (the combination of *L. paracasei* DSM 13434, *L. plantarum* DSM 15312 and DSM 15313) increases measures of trabecular and cortical bone at the unoperated knee joint at tibial epiphyseal bone and at the unoperated and operated knee joints at the femoral epiphyseal bone. The finding that cortical bone is increased with this probiotic treatment supports earlier findings described in WO 2014/163568 and Ohlsson et al (2014, PLoS One, 9(3):e92368). The finding that trabecular bone is increased with this probiotic treatment is surprising in view of the non-observance of an effect on trabecular bone in the study described in WO 2014/163568 and Ohlsson et al (2014, PLoS One, 9(3):e92368).

Epiphyseal trabecular bone analysis (Fig. 2-5) shows that administration of probiotics can a) increase BV/TV and Tb.Th in operated and unoperated knees , b) decrease Tb.Pf in unoperated knees, c) increase medial and lateral bone plate thickness in DMM-operated knees and d) increase medial bone plate thickness in unoperated knees. Although higher epiphyseal BV/TV and increased bone plate thickening can sometimes be a sign of more aggressive OA, we believe that the bone thickening observed in the present study is due to the impact of probiotics on bone formation (similarly to that described for cortical bone in Ohlsson *et al,* 2014, *supra*), rather than the result of more severe OA, particularly since the OA score is lower in the group receiving the probiotic treatment.

Probiotic treatment also provided protection against DMM-induced cartilage damage at the knee joint, particularly at the medial side and at the femur, most particularly at the medial femur (medial femoral condyle). Destabilisation of the *medial* (rather than the lateral) meniscus may have contributed to a bias towards greater overall cartilage damage on the *medial* side, with the effect that the protection resulting from probiotic treatment was more readily identifiable.

Probiotic treatment further provided protection against localised inflammation at the knee joint resulting from DMM.

Therefore, *Lactobacillus* administration, particularly *Lactobacillus paracasei* DSM 13434 in combination with *Lactobacillus plantarum* DSM 15312 and DSM 15313, is useful for the prevention and/or treatment of osteoarthritis in a mammal, particularly by treating and/or preventing cartilage damage and treating and/or preventing inflammation associated with osteoarthritis, particularly inflammation localised to a joint.

### Experimental example 2 - mouse chemokine analyses

We analysed the release of several cytokines/chemokines in mouse serum from Experimental example 1.

### Materials and methods

Millipore's MILLlPLEX^{®} MAP Mouse Cytokine / Chemokine Magnetic Bead Panel was used to assess the presence of inflammatory cytokines in mouse serum samples from the three interventions of the microbiome experiment in Experimental example 1:
**Group 1:** "Sham FMT/glycerol" (mice that had no faecal microbiota transplantation and only administration of vehicle [glycerol]);
**Group 2:** "FMT/glycerol" (mice that had faecal microbiota transplantation followed by administration of vehicle [glycerol]);
**Group 3:** "FMT/probiotics" (mice that had faecal microbiota transplantation followed by administration of probiotics).

The following 25 mouse cytokines were assessed: Granulocyte-colony stimulating factor (G-CSF), granulocyte-macrophage colony-stimulating factor (GM-CSF), interferon gamma (IFNgamma), interleukin 1 alpha (IL-1alpha), interleukin 1 beta (IL-1Beta), interleukin 2 (IL-2), interleukin 4 (IL-4), interleukin 5 (IL-5), interleukin 6 (IL-6), interleukin 7 (IL-7), interleukin 9 (IL-9), interleukin 10 (IL-10), subunit beta of interleukin 12 (IL-12(p40)), interleukin 12 heterodimer (IL12(p70)), interleukin 13 (IL-13), interleukin 15 (IL-15), interleukin 17 (IL-17), interferon gamma-induced protein 10 (IP-10), keratinocyte-derived chemokine (KC), monocyte chemoattractant protein 1 (MCP-1), macrophage inflammatory protein 1-alpha (MIP-1alpha), macrophage inflammatory protein 1-beta (MIP-1beta), macrophage inflammatory protein 2 (MIP-2), RANTES and tumor necrosis factor alpha (TNFalpha).

### Statistical methods

Significant differences between groups were assessed using one-way analysis of variance (ANOVA). The significance level was set at p < 0.05.

Effect size was calculated according to D. B. Wilson, Practical Meta-Analysis Effect Size Calculator [Online Calculator], retrieved 30 March 2020 from https://campbellcollaboration.org/research-resources/effect-size-calculator.html, using the "F-test, 3 or more groups" test for "Standardized Mean Difference (d)".

### Results

Eight of the 25 mouse cytokines that were assessed had a concentration that was below the range of the test, and hence were not analysed: GM-CSF, IFNgamma, IL-1Beta, IL-2, IL-4, IL-7, IL12(p70), TNFalpha.

The results for the remaining 17 mouse cytokines are shown in Fig. 9.

### Reduction in KC with probiotic administration

Fig. 9 shows that out of the 17 mouse cytokines investigated, only KC (keratinocyte-derived cytokine or keratinocyte chemoattractant chemokine, also known as CXCL1) was reduced in mice with reconstituted microbiome and probiotics administration compared to sham control mice (*p* = 0.03).

The independent effect size analysis showed a Cohen's d value of -1.126 for KC (95% confidence interval -2.0531 to -0.1988). Cohen's d values of greater than ±0.8 are considered to indicate a large effect size. Hence, the KC chemoattractant has a large effect size in this experiment.

### Conclusions

There was a reduced release of keratinocyte-derived chemokine (KC) following the probiotic treatment.

KC is a small cytokine from the CXC chemokine family and is also known as chemokine (C-X-C motif) ligand 1, abbreviated to CXCL1, which in humans is encoded by the gene *Cxcl1.* It is produced by chondrocytes and contributes to the pathophysiology of osteoarthritis. KC is highly responsive to mechanical, inflammatory and metabolic stresses (Chauffier et al, 2012, Joint Bone Spine 79(6):604-609). KC is one of the chemokines that is typically increased after injury and which could contribute to the development of inappropriate inflammatory cycles after injury.

Sub-synovial inflammation (Fig. 8) represents the infiltration of inflammatory cells (neutrophils, macrophages and/or lymphocytes). As KC is a neutrophil chemoattractant, the reduction in the sub-synovial inflammation in Fig. 8 seems related to the reduction in KC levels in the probiotic treated mice shown in Fig. 9.

## Claims

1. A combination of live *Lactobacillus* strains, for use in the treatment and/or prevention of osteoarthritis in a mammal, wherein the combination of live *Lactobacillus* strains is *Lactobacillus paracasei* DSM 13434 (8700:2) and at least one strain of *Lactobacillus plantarum,* and wherein the treatment and/or prevention of osteoarthritis is by treating and/or preventing cartilage damage.

2. The combination for use according to Claim 1, wherein the treatment and/or prevention of osteoarthritis is further by treating and/or preventing bone damage at a joint.

3. The combination for use according to Claims 1 or 2, wherein the treatment and/or prevention of osteoarthritis is further by treating and/or preventing inflammation associated with osteoarthritis, preferably by treating and/or preventing inflammation localised to a joint.

4. The combination for use according to any one of Claims 1-3, wherein the mammal is a human.

5. The combination for use according to any one of Claims 1-4, wherein the at least one strain of *Lactobacillus plantarum* is chosen from *Lactobacillus plantarum* DSM 6595 (299), *Lactobacillus plantarum* DSM 9843 (299v^{®}), *Lactobacillus plantarum* DSM 15312 (HEAL 9), *Lactobacillus plantarum* DSM 15313 (HEAL 19), *Lactobacillus plantarum* DSM 15316 (HEAL 99), *Lactobacillus plantarum* DSM 32131 (GOS42), *Lactobacillus plantarum* DSM 17852 (LB3e) and *Lactobacillus plantarum* DSM 17853 (LB7c).

6. The combination for use according to Claim 5, wherein the combination of live *Lactobacillus* strains is *Lactobacillus paracasei* DSM 13434 (8700:2) in combination with *Lactobacillus plantarum* DSM 15312 (HEAL 9) and *Lactobacillus plantarum* DSM 15313 (HEAL 19).

7. The combination for use according to any one of Claims 1-6, wherein the combination is to be administered at least once a day or at least every two, three, four, five, six or seven days, or at least one, two, three, four, five, six or seven times a week.

8. The combination for use according to any one of Claims 1-7, wherein the combination is from about 10⁶ to about 10¹⁴ colony forming units (CFU) per dose, preferably from about 10⁸ to about 10¹² CFU per dose, or more preferably from about 10⁹ to about 10¹¹ CFU per dose, most preferably wherein the combination is about 10¹⁰ CFU per dose.

9. The combination for use according to any one of Claims 1-8, wherein the combination is to be administered as one or more doses in one day, and wherein the daily dose of the combination is from about 10⁶ to about 10¹⁴ CFU per day, preferably from about 10⁸ to about 10¹² CFU per day, or more preferably from about 10⁹ to about 10¹¹ CFU per day, most preferably wherein the daily dose of the combination is 10¹⁰ CFU per day.

10. The combination for use according to any one of Claims 1-9, wherein the combination is present in a composition comprising one or more carriers.

11. The combination for use according to Claim 10, wherein the one or more carriers is/are selected from a pharmaceutically acceptable carrier, a pharmaceutically acceptable excipient, a food-grade carrier, a food-grade excipient, a diluent, and a food.

12. The combination for use according to Claim 11, wherein:
(a) the composition is provided in the form of a solution, suspension, emulsion, tablet, granule, powder, capsule, lozenge, chewing gum, or suppository; or
(b) the food is a cereal-based product, a dairy product, a juice drink, or a fermented food.

13. The combination for use according to any one of Claims 10-12, wherein the composition is supplemented with a chondroprotector including but not limited to chondroitin sulphate, glucosamine, hyaluronic acid.

14. The combination for use according to any one of Claims 1-13, wherein the treatment and/or prevention of osteoarthritis is further by treating and/or preventing sub-synovial inflammation in a joint, preferably localised to the knee joint.

15. The combination for use according to any one of Claims 1-14, wherein the combination is present in a pharmaceutical composition comprising one or more pharmaceutically acceptable excipients, preferably wherein the mammal is a human.

## Patentansprüche

1. Kombination von lebenden *Lactobacillus*-Stämmen zur Verwendung bei der Behandlung und/oder Vorbeugung von Osteoarthritis bei einem Säugetier, wobei die Kombination von lebenden *Lactobacillus*-Stämmen *Lactobacillus paracasei* DSM 13434 (8700:2) und mindestens ein Stamm von *Lactobacillus plantarum* ist, und wobei die Behandlung und/oder Vorbeugung von Osteoarthritis durch Behandlung und/oder Vorbeugung von Knorpelschäden erfolgt.

2. Kombination zur Verwendung nach Anspruch 1, wobei die Behandlung und/oder Vorbeugung von Osteoarthritis ferner durch die Behandlung und/oder Vorbeugung von Knochenschäden an einem Gelenk erfolgt.

3. Kombination zur Verwendung nach Anspruch 1 oder 2, wobei die Behandlung und/oder Vorbeugung von Osteoarthritis ferner durch die Behandlung und/oder Vorbeugung einer mit Osteoarthritis verbundenen Entzündung erfolgt, vorzugsweise durch die Behandlung und/oder Vorbeugung einer auf ein Gelenk beschränkten Entzündung.

4. Kombination zur Verwendung nach einem der Ansprüche 1-3, wobei das Säugetier ein Mensch ist.

5. Kombination zur Verwendung nach einem der Ansprüche 1-4, wobei der mindestens eine Stamm von *Lactobacillus plantarum* ausgewählt ist aus *Lactobacillus plantarum* DSM 6595 (299), *Lactobacillus plantarum* DSM 9843 (299v^{®}), *Lactobacillus plantarum* DSM 15312 (HEAL 9), *Lactobacillus plantarum* DSM 15313 (HEAL 19), *Lactobacillus plantarum* DSM 15316 (HEAL 99), *Lactobacillus plantarum* DSM 32131 (GOS42), *Lactobacillus plantarum* DSM 17852 (LB3e) und *Lactobacillus plantarum* DSM 17853 (LB7c).

6. Kombination zur Verwendung nach Anspruch 5, wobei die Kombination von lebenden *Lactobacillus*-Stämmen *Lactobacillus paracasei* DSM 13434 (8700:2) in Kombination mit *Lactobacillus plantarum* DSM 15312 (HEAL 9) und *Lactobacillus plantarum* DSM 15313 (HEAL 19) ist.

7. Kombination zur Verwendung nach einem der Ansprüche 1-6, wobei die Kombination mindestens einmal täglich oder mindestens alle zwei, drei, vier, fünf, sechs oder sieben Tage oder mindestens ein-, zwei-, drei-, vier-, fünf-, sechs- oder siebenmal pro Woche verabreicht werden soll.

8. Kombination zur Verwendung nach einem der Ansprüche 1-7, wobei die Kombination etwa 10⁶ bis etwa 10¹⁴ koloniebildende Einheiten (KBE) pro Dosis, vorzugsweise etwa 10⁸ bis etwa 10¹² KBE pro Dosis oder noch bevorzugter etwa 10⁹ bis etwa 10¹¹ KBE pro Dosis beträgt, am meisten bevorzugt, wobei die Kombination etwa 10¹⁰ KBE pro Dosis beträgt.

9. Kombination zur Verwendung nach einem der Ansprüche 1-8, wobei die Kombination als eine oder mehrere Dosen an einem Tag verabreicht werden soll und wobei die Tagesdosis der Kombination etwa 10⁶ bis etwa 10¹⁴ KBE pro Tag, vorzugsweise etwa 10⁸ bis etwa 10¹² KBE pro Tag oder noch bevorzugter etwa 10⁹ bis etwa 10¹¹ KBE pro Tag beträgt, wobei die Tagesdosis der Kombination am meisten bevorzugt 10¹⁰ KBE pro Tag beträgt.

10. Kombination zur Verwendung nach einem der Ansprüche 1-9, wobei die Kombination in einer Zusammensetzung vorliegt, umfassend einen oder mehrere Träger.

11. Kombination zur Verwendung nach Anspruch 10, wobei der eine oder die mehreren Träger ausgewählt ist/sind aus einem pharmazeutisch verträglichen Träger, einem pharmazeutisch verträglichen Hilfsstoff, einem lebensmitteltauglichen Träger, einem lebensmitteltauglichen Hilfsstoff, einem Verdünnungsmittel und einem Lebensmittel.

12. Kombination zur Verwendung nach Anspruch 11, wobei:
(a) die Zusammensetzung in Form einer Lösung, Suspension, Emulsion, Tablette, eines Granulats, Pulvers, einer Kapsel, eines Lutschtabletts, eines Kaugummis oder eines Zäpfchens bereitgestellt wird; oder
(b) das Lebensmittel ein Produkt auf Getreidebasis, ein Milchprodukt, ein Saftgetränk oder ein fermentiertes Lebensmittel ist.

13. Kombination zur Verwendung nach einem der Ansprüche 10-12, wobei die Zusammensetzung mit einem Chondroprotektor, einschließlich, aber nicht beschränkt auf Chondroitinsulfat, Glucosamin, Hyaluronsäure, ergänzt ist.

14. Kombination zur Verwendung nach einem der Ansprüche 1-13, wobei die Behandlung und/oder Vorbeugung von Osteoarthritis ferner durch die Behandlung und/oder Vorbeugung einer sub-synovialen Entzündung in einem Gelenk, vorzugsweise lokalisiert im Kniegelenk, erfolgt.

15. Kombination zur Verwendung nach einem der Ansprüche 1-14, wobei die Kombination in einer pharmazeutischen Zusammensetzung vorliegt, umfassend einen oder mehrere pharmazeutisch verträgliche Hilfsstoffe, wobei das Säugetier vorzugsweise ein Mensch ist.

## Revendications

1. Combinaison de souches vivantes de *Lactobacillus,* pour une utilisation dans le traitement et/ou la prévention d'arthrose chez un mammifère, dans laquelle la combinaison de souches vivantes de *Lactobacillus* est *Lactobacillus paracasei* DSM 13434 (8700:2) et au moins une souche de *Lactobacillus plantarum,* et dans laquelle le traitement et/ou la prévention d'arthrose s'effectue en traitant et/ou en prévenant des lésions cartilagineuses.

2. Combinaison pour une utilisation selon la revendication 1, dans laquelle le traitement et/ou la prévention d'arthrose s'effectue en outre en traitant et/ou en prévenant des lésions osseuses au niveau d'une articulation.

3. Combinaison pour une utilisation selon les revendications 1 ou 2, dans laquelle le traitement et/ou la prévention d'arthrose s'effectue en outre en traitant et/ou en prévenant une inflammation associée à l'arthrose, de préférence en traitant et/ou en prévenant une inflammation localisée dans une articulation.

4. Combinaison pour une utilisation selon l'une quelconque des revendications 1 à 3, dans laquelle le mammifère est un être humain.

5. Combinaison pour une utilisation selon l'une quelconque des revendications 1 à 4, dans laquelle la au moins une souche de *Lactobacillus plantarum* est choisie parmi *Lactobacillus plantarum* DSM 6595 (299), *Lactobacillus plantarum* DSM 9843 (299v^{®}), *Lactobacillus plantarum* DSM 15312 (HEAL 9), *Lactobacillus plantarum* DSM 15313 (HEAL 19), *Lactobacillus plantarum* DSM 15316 (HEAL 99), *Lactobacillus plantarum* DSM 32131 (GOS42), *Lactobacillus plantarum* DSM 17852 (LB3e) et *Lactobacillus plantarum* DSM 17853 (LB7c).

6. Combinaison pour une utilisation selon la revendication 5, dans laquelle la combinaison de souches vivantes de *Lactobacillus* est *Lactobacillus paracasei* DSM 13434 (8700:2) en combinaison avec *Lactobacillus plantarum* DSM 15312 (HEAL 9) et *Lactobacillus plantarum* DSM 15313 (HEAL 19).

7. Combinaison pour une utilisation selon l'une quelconque des revendications 1 à 6, dans laquelle la combinaison doit être administrée au moins une fois par jour ou au moins tous les deux, trois, quatre, cinq, six ou sept jours, ou au moins une, deux, trois, quatre, cinq, six ou sept fois par semaine.

8. Combinaison pour une utilisation selon l'une quelconque des revendications 1 à 7, dans laquelle la combinaison est d'environ 10⁶ à environ 10¹⁴ unités formant colonie (UFC) par dose, de préférence d'environ 10⁸ à environ 10¹² UFC par dose, ou plus préférablement d'environ 10⁹ à environ 10¹¹ UFC par dose, dans laquelle la combinaison est de manière la plus préférée d'environ 10¹⁰ UFC par dose.

9. Combinaison pour une utilisation selon l'une quelconque des revendications 1 à 8, dans laquelle la combinaison doit être administrée sous forme d'une ou plusieurs doses par jour, et dans laquelle la dose quotidienne de la combinaison est d'environ 10⁶ à environ 10¹⁴ UFC par jour, de préférence d'environ 10⁸ à environ 10¹² UFC par jour, ou plus préférablement d'environ 10⁹ à environ 10¹¹ UFC par jour, dans laquelle la dose quotidienne de la combinaison est de manière la plus préférée de 10¹⁰ UFC par jour.

10. Combinaison pour une utilisation selon l'une quelconque des revendications 1 à 9, dans laquelle la combinaison est présente dans une composition comprenant un ou plusieurs supports.

11. Combinaison pour une utilisation selon la revendication 10, dans laquelle les un ou plusieurs supports sont choisis parmi un support pharmaceutiquement acceptable, un excipient pharmaceutiquement acceptable, un support de qualité alimentaire, un excipient de qualité alimentaire, un diluant, et un aliment.

12. Combinaison pour une utilisation selon la revendication 11, dans laquelle :
(a) la composition est fournie sous la forme d'une solution, d'une suspension, d'une émulsion, d'un comprimé, d'un granulé, d'une poudre, d'une gélule, d'une pastille, d'un chewing-gum, ou d'un suppositoire ; ou
(b) l'aliment est un produit à base de céréales, un produit laitier, une boisson à base de jus, ou un aliment fermenté.

13. Combinaison pour une utilisation selon l'une quelconque des revendications 10 à 12, dans laquelle la composition est complétée par un chondroprotecteur comportant, mais sans s'y limiter, du sulfate de chondroïtine, de la glucosamine, de l'acide hyaluronique.

14. Combinaison pour une utilisation selon l'une quelconque des revendications 1 à 13, dans laquelle le traitement et/ou la prévention d'arthrose s'effectue en outre en traitant et/ou en prévenant une inflammation sous-synoviale dans une articulation, de préférence localisée dans l'articulation du genou.

15. Combinaison pour une utilisation selon l'une quelconque des revendications 1 à 14, dans laquelle la combinaison est présente dans une composition pharmaceutique comprenant un ou plusieurs excipients pharmaceutiquement acceptables, de préférence dans laquelle le mammifère est un être humain.
